# EUROPÄISCHE PATENTSCHRIFT

(11) **EP 0 773 293 B1**
(45) Veröffentlichungstag und Bekanntmachung des Hinweises auf die Patenterteilung: **03.12.2003**
(21) Anmeldenummer: 96117448.9
(22) Anmeldetag: 30.10.1996
(51) Int. Cl.: C12N 15/12, C12N 9/12, C12N 9/16, C12N 5/10, C12Q 1/42

(54) **Verwendung gentechnisch veränderter tumorigener Zellinien für die Testung von Antitumor-Wirkstoffen**
Use of genetically engineered tumour cell lines in the screening of antitumour-compositions
Applications des Lignées cellulaires tumorales modifiées par génie génétique pour tester des agents antitumoraux

(30) Priorität: 10.11.1995 DE 19542051
(43) Veröffentlichungstag der Anmeldung: 14.05.1997
(73) Patentinhaber: Zentaris GmbH, 60314 Frankfurt/Main (DE); ASTA Medica Oncology GmbH & Co. KG, 60314 Frankfurt/Main (DE)
(72) Erfinder: Beckers, Thomas, Dr., 60596 Frankfurt (DE); Klenner, Thomas, Dr., 55218 Ingelheim (DE); Baasner, Silke, 63456 Hanau (DE)

(56) Entgegenhaltungen:
- DE-A- 4 325 699
- EFRAT S ET AL.: "Conditional transformation of a pancreatic beta-cell line derived from transgenic mice expressing a tetracycline-regulated oncogene" PROCEEDINGS OF THE NATIONAL ACADEMY OF SCIENCES OF USA., Bd. 92, Nr. 8, 11. April 1909, Seiten 3576-3580, XP002082526 WASHINGTON US
- GREGORY B ET AL: "A fast and sensitive colorimetric assay for IL-6 in hepatoma cells based on the production of a secreted form of alkaline phosphatase (SEAP)" JOURNAL OF IMMUNOLOGICAL METHODS, Bd. 170, Nr. 1, 29. März 1994, Seiten 47-56, XP000601567
- BAASNER S ET AL.: "Reversible tumorigenesis in mice by conditional expression of the HER2/c-erbB2 receptor tyrosine kinase" ONCOGENE, Bd. 13, Nr. 5, 5. September 1996, Seiten 901-911, XP002082527 ISSN:0950-9232

## Beschreibung

Die Erfindung betrifft die Verwendung von Vektoren, deren Sequenz ein für ein Reporterprotein kodierendes Gen umfaßt, und mit den Vektoren gentechnisch veränderten tumorigenen Zellinien für die Testung von Antitumor-Wirkstoffen.

Reportergene kodieren für einfach und in geringsten Mengen noch nachweisbare Proteine, die zumeist eine enzymatische Aktivität besitzen. Sie werden gentechnologisch bevorzugt in funktionalen Testsystemen für Untersuchungen regulatorischer genetischer Elemente eingesetzt (Rosenthal, Meth. Enzym. 152, 704-720, 1987), haben sich aber auch für andere Anwendungen, insbesonders Testsystemen für Hochdurchsatz-Screening Systeme in der pharmazeutischen Industrie, hervoragend bewährt. Das klassische Reportergen ist die Chloramphenicol-Acetyltransferase (CAT; Gorman et al., Mol. Cell. Biol. 2, 1044-1051, 1982). Es wurde aber weitestgehend durch andere Reportergene ersetzt. Zu nennen sind hier β-Galaktosidase (An et al., Mol. Cell. Biol. 2, 1628-1632, 1982), Photinus pyralis Luciferase (DeWet et al. Mol. Cell. Biol. 7, 725-737, 1987) und "green fluorescent protein" (GFP; Chalfie et al., Science 263, 802-805, 1994). Den genannten Reporterproteinen ist gemeinsam, daß sie intrazellulär exprimiert werden. Für viele Anwendungen ist dagegen eine sezernierte Expression vorteilhaft. Als sezernierte Reporterproteine sind in der Literatur die humane Placenta-spezifische alkalische Phosphatase (SEAP; Berger et al. Gene 66, 1-10, 1988) und eine sezernierte Luciferase aus Vargula hilgendorfii (Thompson et al., Proc. Natl. Acad. Sci. USA 86, 6567-6571,1989, Thompson et al., Gene 96, 257-262, 1990) beschrieben worden. Prinzipiell kann aber auch durch gentechnische Veränderung ein intrazellulär exprimiertes Reporterprotein sezerniert exprimiert werden. Dies wird möglich, wenn entsprechende Signale, beispielsweise in Form einer eukaryontischen Signalsequenz, gentechnisch in das cDNA Gen eingeführt werden.

Alkalische Phosphatasen (APs; E.C. 3.1.3.1) sind ubiquitär vorhandene Enzyme, die in verschiedensten Isoformen vorliegen. Beim Menschen sind vier distinkte genetische Loci vorhanden, die für die gewebsunspezifische AP, sowie die gewebsspezifischen Formen aus der Placenta (PLAP), dem Intestinaltrakt (IAP) und Keimzellen (GCAP) kodieren (Millán, Anticancer Res. 8, 995-1004, 1988). Maligne Erkrankungen sind häufig mit einer Veränderung des AP-Isoenzym-Profils verbunden. So wurde von Fishman et al. bereits 1968 in Patienten mit Lungenkrebs eine unphysiologisch hohe Aktivität an PLAP im Serum und Krebsgewebe nachgewiesen (Fishman et al., Cancer Res. 28, 150-154, 1968). Als Tumormarker wurde PLAP auch bei Hodenkrebs (Lange et al., Cancer Res. 42, 3244-3247, 1982) sowie bei benignen und malignen Neoplasien des Ovars beschrieben (Nouwen et al., Cancer Res. 45, 892-902, 1985). Dabei besteht aber kein kausaler Zusammenhang zwischen der malignen Entartung und der Expression von alkalischer Phosphatase, speziell der PLAP. Dies konnte durch enzymhistochemische Färbungen für hitzestabile AP in normalem und malignem Cervixgewebe (Nozawa et al., in: Alkaline Phosphatases, 223-234, Alan R. Liss Inc., New York 1984), als auch in Experimenten mit humanen Zellhybriden gezeigt werden (Stanbridge et al. Proc. Natl. Acad. Sci. USA 79, 6242-6245, 1982).

Die cDNA kodierend für PLAP konnte kloniert werden (Millán, J. Biol. Chem. 261, 3112-3115, 1986) und kodiert für ein vermutlich über eine Phosphatidylinositol-Glykan Verknüpfung membranständig exprimiertes Enzym. Es unterscheidet sich durch eine ausgeprägte Hitzestabilität und fehlende Hemmbarkeit durch den Inhibitor Homoarginin von den anderen APs. Dies ermöglicht auch eine sinnvolle Nutzung der PLAP für gentechnische Experimente, insbesonders die Verwendung in modifizierter Form als Reportergen. Von J. Berger et al. wurde erstmals eine gentechnisch verkürzte Form der PLAP beschrieben, die von Zellen in sezernierter Form exprimiert wird und als SEAP bezeichnet wurde (Berger et al. Gene 66, 1-10, 1988; EP 0 327 960 A1. Von der KB Zelllinie, die aus einem humanen Karzinom der Mundschleimhaut etabliert wurde, wird eine hitzestabile Phosphatase sezemiert, die ein Heterodimer aus PLAP und IAP darstellt (Luduena & Sussman, J. Biol. Chem. 251, 2620-2628, 1976; Kodama et al., Biochem. Biophys. Acta 1218, 163-172, 1994). Die durch gentechnische Modifizierung sezemierte Placenta-spezifische alkalische Phosphatase (SEAP) ist als Reportergen insbesondere für Studien der eukaryontischen Genexpression verwendet worden (Cullen & Malim, Meth. Enzym. 216, 362-368, 1992; Jones et al. Oncogene 6, 745-751, 1991).

Die Untersuchung von Substanzen auf anti-proliferative Aktivität erfolgt an in-vitround in-vivo-Testsystemen. Eine breite Testung in einem Eingangsscreening in-vitro erfolgt dabei zumeist an etablierten Tumorzelllinien oder an Modellzelllinien, die durch Transfektion mit einem transformierenden Gen tumorigen werden. Für die in-vitro-Testung stehen eine Vielzahl etablierter Tumorzelllinien zur Verfügung (Alley et al., Cancer Res. 48, 589-601, 1988), wobei zumeist unter Verwendung eines einfachen Testes ein zellulärer Parameter wie beispielsweise die Stoffwechselaktivität oder DNA-Synthese quantifiziert wird. Ein einfacher Test, der die zelluläre Dehydrogenase-Aktivität bestimmt, steht in Form des XTT-Assays zur Verfügung (Scudiero et al., Cancer Res. 48, 4827-4833, 1988).

Ein weiteres System zur Identifizierung von neuen Wirkstoffen, die spezifische Signaltransduktionswege in Säugerzellen beeinflussen, wurde 1991 von Jones et al. (1991) Oncogene, Vol. 6, 745-751 beschrieben. Hierbei werden Vektorsysteme verwendet, welche spezifische respondierende Elemente wie TPA response element (TRE), serum response element (SRE) oder cyclic AMP response element (CRE) fusioniert mit dem SEAP-Gen beinhalten. Nach stabiler Transfektion in Säugerzellen führt dies zu einer Erhöhung der Aktivität von alkaliner Phosphatase nach Stimulation durch TPA, PDGF oder Forskolin. Diese Zelllinien können zur in-vitro Charakterisierung bereits existierender Inhibitoren und zur Auffindung neuer Wirkstoffe, die die spezifischen Signaltransduktionswege beeinflussen, verwendet werden.

Alternativ haben zellfreie rekombinante Testsysteme insbesonders im Hochdurchsatz-Screening für die Auffindung target-spezifischer Tumorpharmaka zunehmend Bedeutung erlangt. Beispielhaft sei hier die Verwendung von rekombinant hergestellten Rezeptorproteinen oder Signalkopplungsproteinen in ELISA-Tests zur Auffindung von Rezeptorantagonisten genannt. Selbst wenn durch eine wohlüberlegte Screening-Hierarchie die Anzahl von Leitstrukturen begrenzt werden kann, ist die Testung in einem validen Tumormodell in-vivo, beispielsweise einem Xenotransplantat auf der Nacktmaus, ein unverzichtbarer Bestandteil der präklinischen Testung.

Die in-vivo Testung an soliden Tumoren erfolgt für Xenotransplantate humaner Tumore oftmals in der Nacktmaus oder der Scid ("severe combined immune deficiency")-Maus. In beiden Fällen handelt es sich um immundefiziente Mausstämme, die die transplantierten Tumorzellen tolerieren. Die klassische Testung von Antitumor-Pharmaka in Nacktmäusen erfolgt durch subkutane Implantation von humanem Tumorgewebe oder Injektion von Zellen einer in-vitro etablierten Tumorzellinie. Stark abhängig von dem implantierten Zellmaterial (Zellzahl, Vitalität, Tumorigenität) bildet sich ein subkutaner Tumor sehr unterschiedlicher Morphologie und Wachstumseigenschaften aus. Dieser kann abhängig von seiner Aggressivität und Invasivität auch zur Ausbildung von Metastasen im Tier führen. Das Wachstum des subkutanen Tumors und damit die Proliferationsrate der ihn bildenden Tumorzellen wird dabei einfach mit einer Schieblehre gemessen oder über Tastung und Vergleich mit einem Plastilin-Modell quantifiziert (Druckrey et al., Drug Res. 6, 539-550, 1956). Dabei wird die Tumorgröße durch das Tumorvolumen (cm³) oder die Tumormasse (g) angegeben. Bei der Behandlung eines Versuchstieres wird die Hemmung des Tumorwachstums oder die Regression des Tumors als kurative Wirkung einer Substanz bezeichnet. Ebenso bietet sich die Verlängerung der Lebensdauer als ein Maß für den heilenden Effekt einer Wirksubstanz an. Dies ist insbesonders bei nicht subkutan wachsenden Tumoren eine relevante Meßgröße. Bei dieser Vorgehensweise werden verschiedene Nachteile offensichtlich, die versuchstechnisch bisher nicht zu vermeiden waren. Wird beispielsweise ein solider, subkutaner Tumor als Folge einer Behandlung in den inneren Zellschichten nekrotisch, ist dies unter Umständen durch Tastung nicht zu detektieren. Führt ein subkutan transplantierter Tumor zu einer Metastasierung in verschiedene Organe, sind diese Metastasen zunächst verborgen und erst durch die Funktionsbeeinträchtigung des befallenen Organs oder nach einer Biopsie sichtbar.

Der vorliegenden Erfindung liegt demnach die Aufgabe zugrunde, ein Tiermodell zu entwickeln, das ermöglicht, die Gesamtzahl vitaler Tumorzellen im Gesamtorganismus unter dem Einfluß einer Therapie ohne Tastung oder Biopsie zu quantifizieren. Insbesondere ist die Erfindung darauf gerichtet, das genannte Tiermodell mittels gentechnischer Veränderung von Zellinien durch geeignete Vektoren zu verwirklichen.

Diese Aufgabe wird nach einer ersten Ausführungsform der Erfindung mit der Verwendung von Vektoren gelöst, die eine Nukleotidsequenz der allgemeinen Formel (I)

R - X - A - X - IRES - X - B - X - polyA (I)

umfassen, in der
- R: eine regulatorische Nukleotidsequenz für eine konstitutive oder induzierbare Genexpression,
- A: ein Gen, kodierend für ein Protein, das ein tumorigenes Wachstum von Zellen induzieren kann,
- IRES: eine Nukleotidsequenz viralen, zellulären oder synthetischen Ursprungs ist, die in der Stufe der Translation für die interne Initiation verantwortlich ist,
- B: ein Gen, kodierend für ein sensitiv detektierbares sekretiertes Protein,
- poly(A): eine Nukleotidsequenz für die Polyadenylierung des Transkriptes und
- X: optionale Linkersequenzen darstellen.

Die Vektoren der allgemeinen Formel (I) werden verwendet, wenn nicht tumorigene Zellinien gentechnisch verändert werden sollen.

Wenn Zellinien gentechnisch verändert werden sollen, die vor der Veränderung bereits tumorigene Eigenschaften aufweisen, wird die obengenannte Aufgabe nach einer zweiten Ausführungsform der Erfindung mit Vektoren gelöst, die eine Nukleotidsequenz der allgemeinen Formel (II)

R - X - B - X - IRES - X - C - X - polyA (II)

umfassen, in der
- R: eine regulatorische Nukleotidsequenz für eine konstitutive oder induzierbare Genexpression,
- B: ein Gen, kodierend für ein sensitiv detektierbares sekretiertes Protein,
- IRES: eine Nukleotidsequenz viralen, zellulären oder synthetischen Ursprungs ist, die in der Stufe der Translation für die interne Initiation verantwortlich ist,
- C: ein Gen, kodierend für ein Protein, das eine Selektion basierend auf einer Resistenz ermöglicht,
- poly(A): eine Nukleotidsequenz für die Polyadenylierung des Transkriptes und
- X: optionale Linkersequenzen darstellen.

Die vorliegende Erfindung beschreibt die Verwendung von dicistronischen Expressionsvektoren, die die Expression eines für ein Reporterprotein kodierenden Gens B mit der eines zweiten Gens A oder C koppeln. Diese Kopplung erfolgt über eine "internal ribosomal entry site" (IRES), die zwischen die beiden zu exprimierenden Gene plaziert wird. Im Unterschied zu den bekannten multicistronischen Expressionseinheiten für die äquimolare Expression von heterodimeren Proteinen beispielsweise PDGF-A/B (Dirks et al., Gene 128, 247-249, 1993; WO 94/05785, wobei dort auch die in Frage kommenden IRES-Sequenzen beschrieben sind), ist erfindungsgemäß eine äquimolare Expression nicht zwingend erforderlich, da das wesentliche funktionale Merkmal die Kopplung der Proteinsynthese des Reporterproteins mit der eines funktional interessanten zweiten Proteins ist.

Gemäß einer bevorzugten Ausführungsform der Erfindung kodiert das Gen B für eine sekretierte alkalische Phosphatase, insbesondere für die sekretierbare humane Placenta-spezifische alkalische Phosphatase (SEAP). Vorteilhaft ist, daß SEAP von transfektierten Zellen sezerniert wird und damit in Kulturüberständen nachweisbar ist. Zudem gibt es einfache, sehr sensitive Nachweisverfahren basierend auf chemoluminometrischen Substraten (Bronstein et al., Anal. Biochem. 219, 169-181, 1994).

Bei Vektoren, die ein zweites Gen A umfassen, kodiert dieses vorzugsweise für zelluläre Transformation. Besonders bevorzugt sind solche Gene A, die für eine Rezeptortyrosinkinase (RTK), die ein tumorigenes Wachstum von Zellen induzieren kann, insbesondere für die Rezeptortyrosinkinase erbB2/HER2, kodieren.

Wenn die Vektoren ein Gen C umfassen, kodiert dieses vorzugsweise für ein Enzym, das eine zelltoxische Verbindung inaktiviert, insbesondere für das Enzym Aminoglykosid-3'-phosphotransferase. Solche Gene C ermöglichen eine Selektion basierend auf einer Resistenz.

Die Vektoren ermöglichen beispielsweise für Vektoren der allgemeinen Formel (I) mit einer Kopplung eines transformierenden Gens mit SEAP, daß dessen Transkription / Expression und damit auch die Anzahl vitaler, transformierter Zellen über die SEAP-Aktivität in Kulturüberständen in-vitro und in-vivo beispielsweise in Serum quantifiziert werden kann. Bei Verwendung von Vektoren dieses Typs wird ein Gen C kodierend beispielsweise für Puromycin-N-acetyltransferase (Vara et al. Nucl. Acids Res. 14, 4617-4624, 1986), Hygromycin-B-phosphotransferase (Blochlinger & Diggelmann, Mol. Cell. Biol. 4, 2929-2931, 1994) oder Amino-glykosid-3'-phosphotransferase (Colbére-Garapin et al. J. Mol. Biol. 150, 1-14, 1981) mittels Kotransfektion in die Zielzelle überführt und ermöglicht eine Selektion auf stabil transfektierte Zellen.

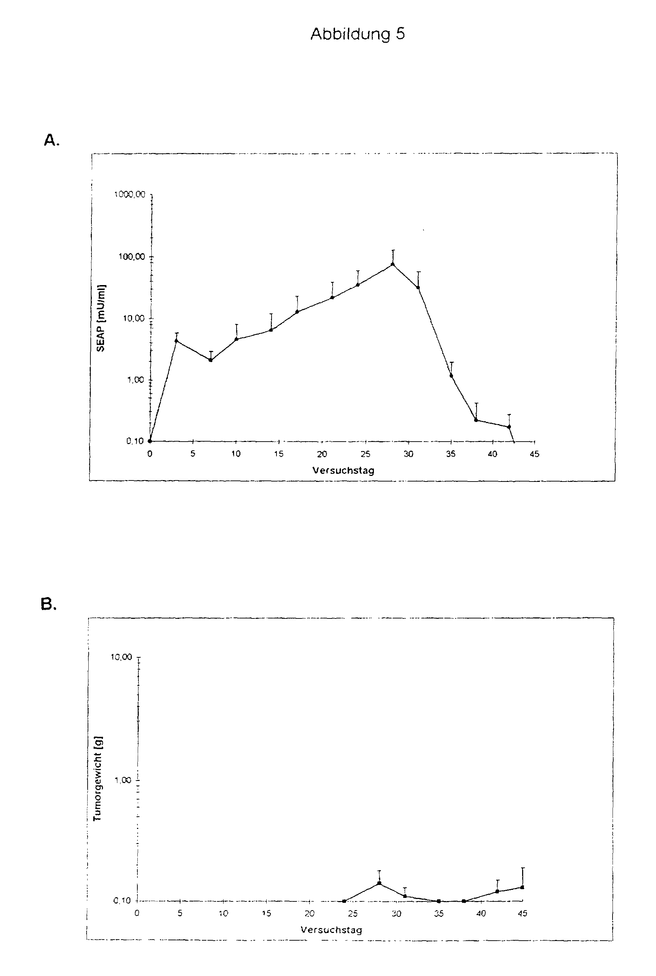

Zur Expression eines Gens in Säugerzellen, wird dieses im Kontext eines entsprechenden Expressionsvektors zur stabilen Transfektion verwendet. Ein solcher Vektor für die Expression in Säugerzellen enthält ein Gen (oder Gene) unter der Kontrolle entsprechender genetischer Elemente (u.a. Promotor, Polyadenylierungssignal), erfindungsgemäß eine regulatorische Nukleotidsequenz R für eine konstitutive oder induzierbare Genexpression und eine Nukleotidsequenz poly(A) für die Polyadenylierung des Transkripts. Das Promotorelement ist entweder konstitutiv aktiv, wobei ein von SV-40 abgeleiteter Promotor ("immediate early promotor" von SV-40) besonders vorteilhaft ist, oder kann mit Tetracyclin oder einem Derivat in seiner Aktivität reguliert werden (Gossen & Bujard, Proc. Natl. Acad. Sci. USA 89, 5547-5551, 1992; WO 94/29442). Letzteres Prinzip ist in-vivo in transgenen Mäusen funktionell (Furth et al. Proc. Natl. Acad. Sci. USA 91, 9302-9306, 1994). Die vorliegende Erfindung weist den Vorteil auf, daß das Prinzip auch in Nacktmäusen für beispielsweise subkutan transplantierte, gentechnisch modifizierte tumorigene Zellen anwendbar ist (siehe Abb.5).

Optionale Linkersequenzen X bestehen aus in Klonierungsvektoren üblichen Linkersequenzen, z. B. Polylinkern, und enthalten z. B. Schnittstellen für Restriktionsendonukleasen.

Häufig bedient man sich der Technik der Kotransfektion, d.h. verschiedene Expressionsvektoren werden zusammen in die Zielzelle überführt. Dies ist insbesonders dann die Methode der Wahl, wenn der Expressionsvektor mit dem (den) zu exprimierenden Gen(en) keine Selektionsgene enthält. Einen allgemeinen Überblick zu dieser Thematik , als auch zu den Methoden der stabilen Transfektion von Säugerzellen, bietet die Monographie von M. Kriegler ("Gene transfer and expression", W.H. Freeman Inc. New York, 1990). Steht das Gen unter der Kontrolle eines regulierbaren Promotorelementes, kann die Expression an- oder abgeschaltet werden. Ein sehr elegantes Tetracyclinreguliertes Genexpressions-System ist das oben bereits erwähnte von Gossen und Bujard. Über dieses induzierbare Expressionssystem kann die Expression eines transformierenden Gens in-vitro und in-vivo an- oder abgeschaltet werden und ist über die SEAP-Aktivität quantifizierbar. Es verwendet ein Fusionsprotein in Form des Tetracyclin-kontrollierten Transaktivators (tTA), welches über Bindung an eine spezifische Tetracyclin-Operatorsequenz einen Minimalpromotor aktiviert. Durch Zugabe von Tetracyclin oder Derivaten wird das tTA Protein von der Operatorsequenz abgelöst und damit die Genexpression abgeschaltet.

Gemäß einer weiteren bevorzugten Ausführungsform der Erfindung umfaßt demnach R eine Tetracyclin-Operatorsequenz und einen Minimalpromotor. Insbesondere stellt R die Nukleotidsequenz tet0₇ / CMV dar.

Erfindungsgemäß werden mit den Vektoren der allgemeinen Formel (I) oder (II) Säugerzellinien stabil transfektiert. Besonders bevorzugt sind transfektierte Säugerzellinien, die Einzelzellklone darstellen. Bevorzugte Beispiele für erfindungsgemäß transfektierte Säugerzellinien sind transfektierte NIH3T3-Fibroblasten. Insbesondere werden mit den Vektoren der allgemeinen Formel (II) Tumorzellinien transfektiert, während mit den Vektoren der allgemeinen Formel (I) andere Säugerzellinien gentechnisch zu Tumorzellinien verändert werden.

Die zur Herstellung der Vektoren notwendigen molekularbiologischen Techniken sind ausführlich in Maniatis et al. ("Molecular cloning: a Laboratory Manual", 2nd edition, Cold Spring Harbor Laboratory / New York, 1992), Ausubel et al ("Current Protocols in Molecular Biology", J. Wiley & Sons) sowie den Patenten von Cohen et al. ("Process for producing biologically functional molecular chimeras (by Genetic engineering)" U.S. Pat. Appl. # 4,237,224) und Collins et al. ("Process for the production of hybrid bacteria", U.S. Pat. Appl. # 4,304,863) beschrieben.

Die Erfindung betrifft weiterhin ein Verfahren zum Screening von Antitumor-Wirkstoffen, das die folgenden Schritte umfaßt:
a) Konstruktion eines wie oben beschriebenen Vektors der allgemeinen Formel (I) oder (II),
b) stabile Transfektion einer Säugerzellinie mit dem in Schritt a) erhaltenen Vektor,
c) Wachsen der in Schritt b) erhaltenen Säugerzellinie in einem Säugergewebe,
d) Entnahme eines Serums aus dem in Schritt c) erhaltenen Säugetiergewebe und
e) Nachweis des Reporterproteins in dem in Schritt d) erhaltenen Serum.

Die vorliegende Erfindung beschreibt den direkten Nachweis von SEAP aus Versuchstieren (Nacktmäusen), die Implantate gentechnisch veränderter Zellen enthalten. Der Nachweis von SEAP erfolgt beispielsweise aus dem Serum der Tiere, das diesen während eines Experimentes wiederholt entnommen wird. Diese mehrmalige Entnahme aus der Unterzungenvene ist nicht mit einer nachhaltigen Beeinflußung des Versuchstieres verbunden. Der Nachweis ist hochsensitiv möglich, wobei zur Messung der SEAP Aktivität ein fotometrischer Enzymtest unter Verwendung des Substrates 4-Nitrophenylphosphat bzw. ein noch wesentlich sensitiverer chemoluminometrischer Nachweis unter Verwendung des Substrates CSPD® (Tropix Inc.; Bronstein et al., Biotechniques 17, 172-177, 1994) eingesetzt wird. Die Hintergrundaktivität an Phosphatasen im Serum ist unter den gewählten Testbedingungen vernachlässigbar klein. Aufgrund der Implantation einer verhältnismäßig großen Zellzahl verbunden mit einem geringen Plasmavolumen kommt es zu einem Konzentrationseffekt. Selbst wenn transfektierte Zellen in-vitro nur geringe Mengen an SEAP in das Kulturmedium sezernieren, ist diese im Serum der Versuchstiere um ein vielfaches konzentriert und hierdurch einfach nachweisbar. Diese Anwendung muß aber nicht auf SEAP beschränkt sein, sondern es eignen sich prinzipiell auch andere Gene kodierend für sezernierte Reporterproteine wie beispielsweise Vargula hilgendorfii Luciferase für die in der Erfindung beschriebene Methode. Hierin eingeschlossen sind auch gentechnisch veränderte cDNA Gene, die - beispielsweise durch Anhängung einer Signalsequenz und/oder Deletion membranständiger Sequenzen - dann für ein sezerniertes Protein kodieren.

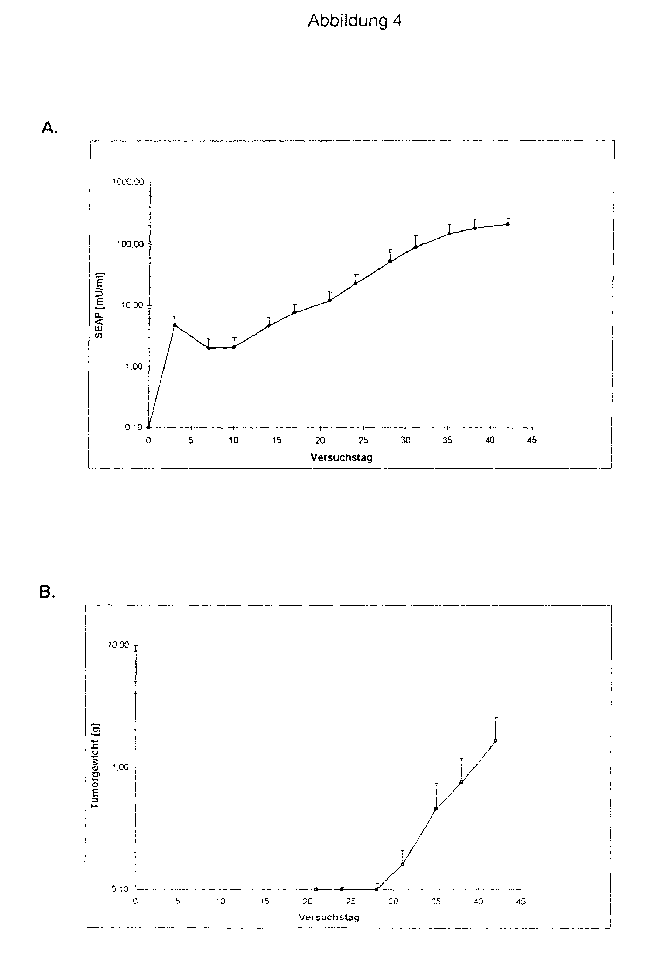

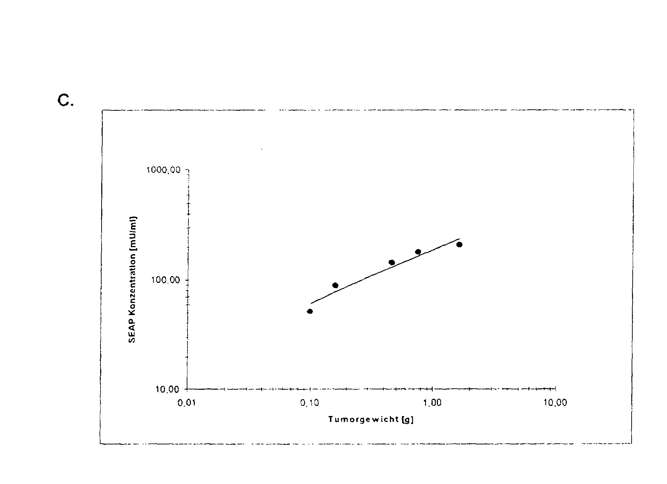

Die vorliegende Erfindung beschreibt ein neuartiges Verfahren zur Bestimmung der Tumormasse ("tumor load"), die über die Messung von SEAP-Aktivität im Serum erfolgt. Diese wird von den Tumor-bildenden, gentechnisch modifizierten Zellen sezerniert und so in den Blutkreislauf überführt. Über die Messung der SEAP-Aktivität in Serum ist die direkte quantitative Erfassung vitaler Tumorzellen möglich. Es zeigt sich eine sehr gute Korrelation mit dem durch Tastung bestimmten Tumorgewicht (siehe hierzu Abb.4). Tumorzellen sind dabei schon im Tier nachweisbar, obwohl noch kein tastbarer Tumor vorliegt. Damit erlaubt die in der vorliegenden Erfindung beschriebene Methode einen hochempfindlichen Nachweis des "tumor-load" in-vivo. Zudem ist sie nicht auf subkutane Tumore beschränkt, sondern ermöglicht auch ein Monitoring von Therapie Experimenten mit metastasierenden oder orthotop transplantierten Tumoren beliebiger Art. Beispielhaft wird der Nachweis orthotop transplantierter Kolonkarzinomzellen in Nacktmäusen über die Messung von SEAP-Aktivität im Serum der Tiere in der vorliegenden Erfindung gezeigt.

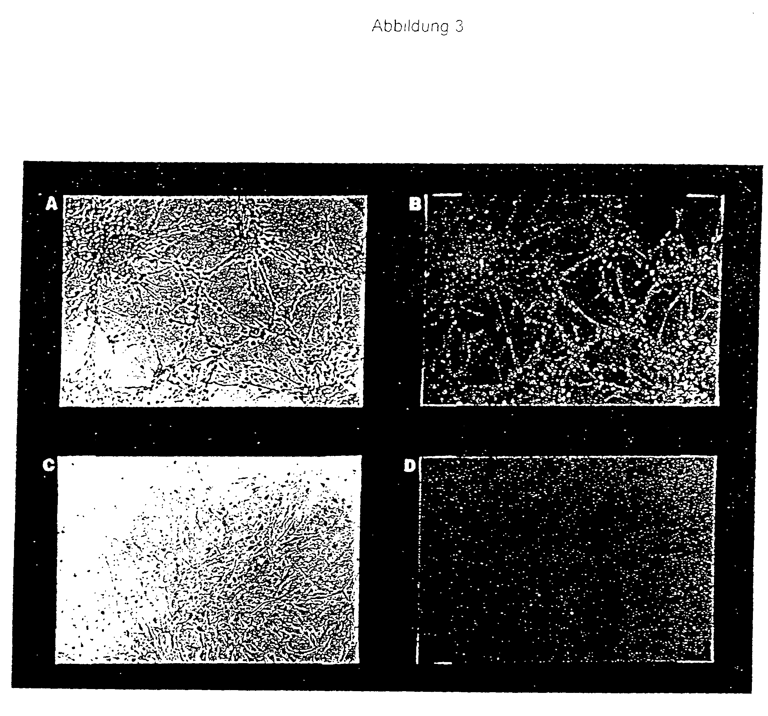

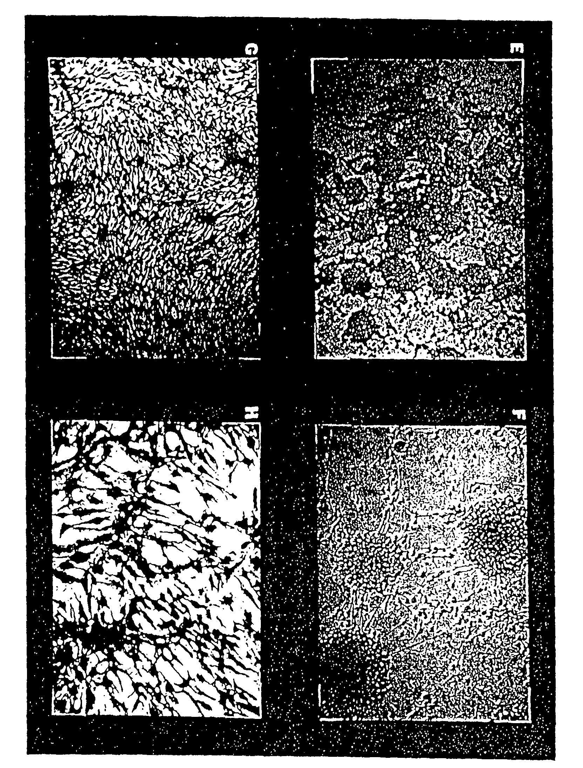

In der vorliegenden Erfindung werden nun Experimente mit gentechnisch veränderten Zellen gezeigt, die dies exakt ermöglichen. In der Erfindung sind in einem Beispiel transfizierte murine NIH3T3 Fibroblasten gezeigt, die durch Überexpression der Rezeptortyrosinkinase HER2 transformiert werden (siehe hierzu Abb. 3 sowie die Literatur von Hudziak et al. Proc. Natl. Acad. Sci. USA 84, 7159-7163, 1987 und DiFiore et al. Science 237, 178-182, 1987). Der Rezeptortyrosinkinase HER2 kommt bei verschiedenen humanen Tumoren nach heutigem Erkenntnisstand eine ganz wesentliche Bedeutung zu (Slamon et al. Science 235, 177-182, 1987). In dem hier beschriebenen Modellsystem ist die HER2-Expression mit der von humaner sekretierter placentarer alkalischer Phosphatase (SEAP) über eine IRES-Sequenz transkriptionell gekoppelt. HER2 überexprimierende, transfektierte NIH3T3 Zellen sind nach allgemein anerkannten Kriterien voll transformiert (siehe hierzu auch Abb.3 und Daten in Tab. 1). Die von diesen tumorigenen Zellen sezernierte SEAP kann in geringsten Mengen im Serum von Versuchstieren nachgewiesen werden (siehe hierzu Abb. 4A). Durch Hitzebehandlung von Serum (30 min bei 65°C) und Zugabe des Inhibitors Homoarginin werden andere im Serum enthaltene hitzelabile Phosphatasen praktisch vollständig inaktiviert. Für die Messung werden nur geringste Mengen an Serum benötigt, beispielsweise 25µl in einem Standardexperiment. Dieses kann während eines Versuches mehrfach in bestimmten Zeitabständen aus der Vena sublingualis entnommen werden, ohne daß das Versuchstier hierdurch nachhaltig beeinflußt wird. Bei einem subkutanen Modelltumor zeigt dabei die durch Tastung bestimmte Tumormasse eine hervorragende Korrelation mit der im Serum der Versuchstiere bestimmten SEAP-Aktivität (siehe hierzu Abb. 4C). In einem anderen Beispiel kann die Expression der Rezeptortyrosinkinase HER2 und damit gekoppelt des Reporterproteins SEAP transkriptionell in-vivo abgeschaltet werden. Auf die Funktionsweise des zugrundeliegenden Prinzips wurde schon eingegangen. Das Ergebnis ist beispielhaft für ein in-vivo Experiment mit einem subkutanen Modelltumor gezeigt (siehe Abb. 5). Kurze Zeit nach der Injektion von Anhydrotetracyclin nimmt die im Serum nachweisbare SEAP-Aktivität mit einer linearen Kinetik ab. Das durch Tastung bestimmte Tumorgewicht bleibt mit etwa 0.1 g bei allen behandelten Tieren konstant, d.h. das Wachstum des Tumors konnte gestopt werden. Die aus diesem Experiment bestimmte Plasmahalbswertszeit für SEAP beträgt etwa 28h. Diese relativ kurze Plasmahalbswertszeit ermöglicht es, daß sich eine Abnahme vitaler Tumorzellen in einem Therapieexperiment direkt in einer Erniedrigung der SEAP-Plasmaaktivität ausdrückt. Ein derartiges Therapieexperiment könnte in dem vorliegenden Beispiel der HER2 Rezeptortyrosinkinase in der Applikation eines HER2 RTK-spezifischen Antagonisten (Tyrphostin) vorgenommen werden. Der Ansatz der Inhibition von Proteintyrosinkinasen im allgemeinen und hierzu geeignete Substanzen sind aus der Literatur gut bekannt (Levitzki & Gazit, Science 267, 1782-1788, 1995).

In der Verknüpfung des Tetracyclin-regulierten Expressionssystems mit dicistronischen Vektoren und der Kopplung eines transformierenden Proteins wie HER2 mit SEAP als Reporterprotein, zeigt die vorliegende Erfindung eine neuartige Kombination und Anwendung bereits bekannter Systeme. Über die Messung der SEAP-Aktivität im Serum von Versuchstieren nach Gabe von Tetracyclin oder einem Derivat ist die Funktion des Expressionsystems zu monitoren. Über die Bestimmung des Tumorwachstums mittels Tastung kann zugleich die Relevanz des Tumor-auslösenden Gens für das Wachstum des soliden Tumors beurteilt werden. Dies ist für eine Beurteilung der Bedeutung eines Targets für die Tumortherapie von großer Bedeutung. Bevor geeignete Hemmstoffe des Onkoproteins gefunden sind, kann in dem hier vorgestellten Modell ein transkriptioneller "knock-out" simuliert werden. Durch Hemmung auf Transkriptionsebene kann unter Einbeziehung der biologischen Halbwertszeit des Onkoproteins, dessen Bedeutung in der Tumorentstehung und dem weiteren Wachstum des soliden Tumors modellhaft studiert werden. Auch eine weitere Anwendung sei hier angesprochen. Durch stabile Transfektion eines Onkogens oder durch dessen dysregulierte Expression transformierte Zellen sind genetisch nicht beliebig stabil. Dies gilt insbesonders für murine Fibroblasten wie NIH3T3- oder auch Rat1-Zellen. Bei Verwendung derart gentechnisch veränderter Zellen ist es deshalb sehr wichtig, die Abhängigkeit des transformierten Phänotypus von dem eingebrachten Onkogen kontinuierlich zu überprüfen. Dies ist mit dem in der vorliegenden Erfindung beschriebenen Tetracyclin-kontrollierten Expressionsystem in-vivo hervorragend möglich. Bei Injektion von tumorigenen Zellen und gleichzeitiger Gabe von Anhydrotetracyclin dürfen sich keine Tumore ausbilden, sofern das unter Kontrolle des induzierbaren Promotorelementes plazierte Onkogen allein das tumorigene Wachstum in-vivo induziert. Sind allerdings im Verlauf der in-vitro Kultur weitere genetische Defekte eingetreten ("second hits"), dann bilden sich auch bei supprimierter Expression des transformierenden Gens Tumore in der Nacktmaus aus.

Die vorliegende Erfindung ist aber nicht auf durch Transfektion transformierte Modellzellinien wie NIH3T3 Fibroblasten beschränkt. Vielmehr können beliebige Tumorzellen derart gentechnisch verändert werden, daß sie nachweisbare Mengen an SEAP sezernieren. Seit langem sind Tumormarker im Serum von Krebspatienten für diagnostische Zwecke verwendet worden. Zu nennen wären beispielhaft humanes Chorion Gonadotropin (hCG), saure Phosphatase, Prostata-spezifisches Antigen (PSA), aber auch PLAP. Verschiedene Formen von alkalischer Phosphatase sind in humanen Tumorzellinien nachweisbar, auch hitzestabile Isoenzyme (Benham et al. Int. J. Cancer 27, 637-644, 1981). Die Expression ist aber häufig membranständig oder intrazellulär. Über den Mechanismus der in einigen Fällen zu beobachtenden sezernierten Expression ist offensichtlich nur wenig bekannt. Dieser kann aber mit der für PLAP gefundenen Phosphatidylinositol-Glykan Verknüpfung zusammenhängen. Wie in Tabelle 2 gezeigt ist, ist im Kulturmedium in-vitro bei der Minderheit der ausgewählten Tumorzellinien verschiedenster Herkunft, eine signifikante Aktivität hitzestabiler AP nachweisbar. Beispielhaft ist für drei Tumorzellinien gezeigt, daß diese nach stabiler Transfektion mit einem entsprechenden Expressionsvektor signifikante Mengen an SEAP sezernieren. Selbst wenn eine nicht-transfizierte Tumorzellinie hitzestabile AP Aktivität in das Kulturmedium sezerniert, kann mittels Transfektion die Menge signifikant erhöht werden.

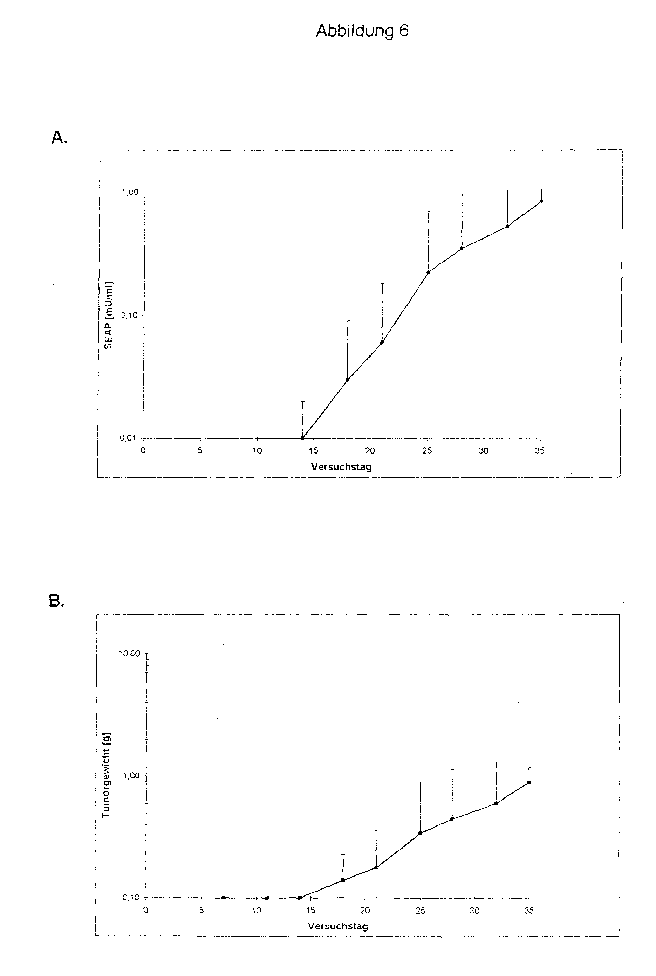

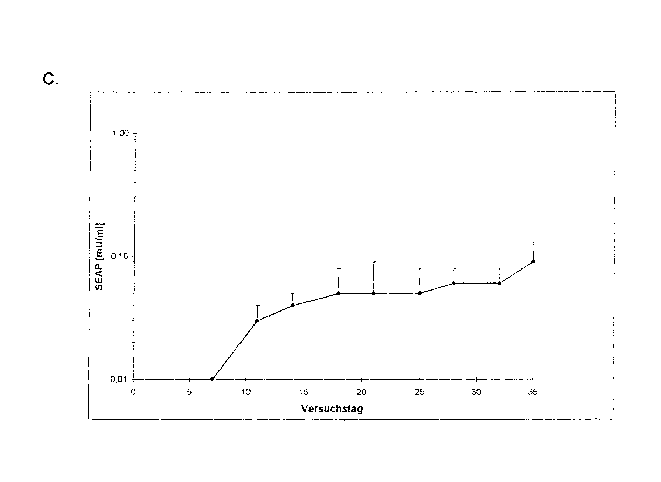

Ein optimales Tumormodell in-vivo berücksichtigt, daß die Krebszellen in einem Tumor in einem komplexen Wechselspiel mit dem Gesamtorganismus, insbesonders dem umgebenden Gewebe, stehen. Beispielhaft seien hier parakrine Wachstumsstimulation, Tumor-Vaskularisierung oder Zell-Matrix-Interaktionen und Metastasierung genannt. In einem subkutanen Tumormodell sind diese komplexen Vorgänge nur sehr unzureichend zu simulieren und häufig mit den physiologischen Bedingungen nicht vergleichbar. Zudem wachsen nur ein Teil der humanen Tumore nach subkutaner Implantation auch wirklich zu einem soliden Tumor heran. Eine methodische Alternative ist in der orthotopen Transplantation von Tumorzellen zu sehen. Hier wird das Tumormaterial direkt in das Ursprungsorgan transplantiert. Die Vorteile orthotop transpantierter Tumore wurden bespielsweise für maligne Melanome, Prostatatumore oder Osteosarkome gezeigt (Kerbel et al. Cancer & Metast. Rev. 10, 201-215, 1991; Stephenson et al. J. Natl. Cancer Inst. 84, 951-957, 1992; Berlin et al. Cancer Res. 53, 4890-4895, 1993). Eine Beurteilung des Erfolges einer Therapie ist bei orthotop transplantierten Tumoren zunächst nur über die Bestimmung der Verlängerung der Lebensdauer möglich. Werden die Tumorzellen jedoch wie oben beschrieben gentechnisch verändert, sodaß diese SEAP synthetisieren, kann das Wachstum der Tumorzellen über Messung von SEAP-Aktivität im Serum der Versuchstiere exakt bestimmt werden. Besonders einfach wird dies, wenn die für die Transplantation verwendeten Tumorzellen selbst schon meßbare Aktivitäten an hitzestabiler Phosphatase sezernieren (siehe Tabelle 2, insbesonders KB-Zellinie). Modellhaft ist ein orthotopes Transplantationsexperiment (Abb. 6C) im Vergleich mit dem subkutanen Tumor (Abb. 6A/B) für die humane, stabil transfektierte Kolon-Adenokarzinom Zellinie HT-29 gezeigt. Unter Verwendung der in der vorliegenden Erfindung gezeigten Methoden, werden somit auch orthotope Tumormodelle einem Routine-Screening auf Antitumor-Pharmaka zugänglich.

Zuletzt sei noch erwähnt, daß die vorliegende Erfindung potentiell zu einem Umdenken in der Durchführung von Tierversuchen mit der Folge einer Reduktion von Versuchstieren führen kann. Die Messung von SEAP-Aktivität im Serum von Versuchstieren ist sehr sensitiv möglich. Aus diesem Grund werden die Tumorzellen quantifizierbar, bevor ein Tumor makroskopisch sichtbar und tastbar ist. Ein Therapieversuch könnte so schon beginnen, bevor ein Tumor Symptome und damit Belastungen verursacht. Der Therapieerfolg kann dann einfach an der Abnahme der SEAP-Aktivität im Serum bestimmt werden. Der belastende Effekt eines soliden Tumors für das Versuchstier wäre somit auf ein Minimum zu beschränken. Zudem könnten multiple Therapiezyklen konzipiert werden, die die normal für einen statistisch auswertbaren Versuch notwendige Anzahl von Versuchstieren entsprechend reduzieren.

Die Anwendung der vorliegenden Erfindung läßt sich wie folgt zusammenfassen.
(i) Modell- oder Tumorzellinien werden derart gentechnisch verändert, daß sie SEAP synthetisieren und sezernieren. Diese ist im Serum von tumortragenden Versuchstieren sensitiv nachweisbar und korreliert mit der Tumormasse. Damit ist es möglich, die Anzahl vitaler Tumorzellen in Versuchstieren über die SEAP Aktivität im Serum zu bestimmen. Dies ermöglicht ein exaktes Monitoring von Therapieexperimenten mit potentiellen Antitumor-Wirkstoffen unter Verwendung beliebiger Tumormodelle.
(ii) Durch die Verwendung dicistronischer Expressionsvektoren mit SEAP als Reportergen sowie eines mit Tetracyclin-regulierbaren Expressionsystems kann die Relevanz eines beliebigen transformierenden Gens für die Tumorentstehung und das Wachstum eines soliden Tumors modellhaft studiert werden. Durch einen transkriptionellen "knock-out" in-vivo kann die Wirkung eines noch aufzufindenen Antagonisten simuliert werden und damit die weitere Strategie wesentlich beeinflußt werden.

Die vorliegende Erfindung wird deshalb bevorzugt im Screening von potentiellen Antitumor-Wirkstoffen in-vivo und in der Target-Validierung vor einem derartigen Screening verwendet werden.

Die Erfindung wird im folgenden anhand von Beispielen näher erläutert, die illustrieren sollen, wie Vektoren mit dem SEAP Gen für die stabile Zelltransfektion eingesetzt werden können und wie mit den Vektoren gentechnisch veränderte Zellen für in-vivo Experimente mit Xenotransplantaten an Nacktmäusen zur sensitiven Bestimmung der Masse vitaler Tumorzellen Verwendung finden. Ebenso soll in Modellexperimenten gezeigt werden, wie die Erfindung für die sensitive Detektion orthotop transplantierter Tumore verwendet wird.

### Beispiel 1: Dicistronische Expressionsvektoren mit SEAP

Nachfolgend sind die für die verschiedenen Experimente verwendeten Expressionsvektoren beschrieben, sowie die für deren Herstellung benötigten Methoden exemplarisch benannt.

### Methoden:

Für die Klonierungsexperimente sind molekularbiologische Standardmethoden eingesetzt worden, die u.a. in Ausubel et al. Current protocols in molecular biology, J. Wiley & Sons 1989 und Maniatis et al. ("Molecular cloning: a Laboratory Manual", 2nd edition, Cold Spring Harbor Laboratory / New York, 1992) detailliert beschrieben sind.

Für die DNA-Analyse werden Restriktionsendonukleasen des Typs II in dem entsprechenden Puffer (beispielsweise 6mM Tris pH 7.5, 6mM MgCl₂, 100mM NaCl, 0.4mg/ml BSA, 0.002% NaN₃) mit 1-10µg Plasmid-DNA bei 37°C inkubiert. Nach einer entsprechenden Inkubationszeit von beispielweise 3h werden die Proben gelelektrophoretisch aufgetrennt.

Diese Auftrennung von DNA Fragmenten nach Fragmentierung durch Restriktionsenzyme erfolgt in Agarosegelen der Konzentration 0.8 - 2% (w:v), abhängig von der Größe der zu analysierenden DNA. Zur Größenbestimmung linearer DNA-Fragmente in Basenpaaren (Bp) wird ein Größenstandard verwendet (beispielsweise λ-DNA BstEII geschnitten). Als Laufpuffer für die Elektrophorese wird TAE Puffer (40mM Tris-Acetat, 1 mM EDTA, 0.5mg/l Ethidiumbromid) eingesetzt. Der Lauf erfolgt bei konstanter Spannung von beispielsweise 200V bei Verwendung einer Gelkammer der Größe 35,5 x 18cm. Nach Beendigung des Laufes wird das Gel mit dem Gelträger entnommen und auf einem Transilluminator unter UV-Anregung die DNA sichtbar gemacht.

Die kovalente Verknüpfung von DNA-Fragmenten wird durch die DNA-Ligase des Phagen T4 katalysiert. Nach Schneiden der DNA mit dem(n) entsprechenden Restriktionsenzym(en) und Auftrennung in einem NuSieve-LMP ("low melting point") Agarose-Gel in TAE-Puffer, wird das gewünschte DNA Fragment aus dem Gel ausgeschnitten und bei 70°C geschmolzen. Ein typischer Ligationsansatz besteht aus 1 µl Vektor-DNA, 2-4µl DNA Fragment(e), 5µl 10x Ligations-Puffer (500mM Tris pH7.5, 100mM MgCl₂, 100mM DTT, 10mM rATP, 250µg/ml BSA) , 1 µl T4 DNA-Ligase (400Units/µl) in 50µl Gesamtvolumen. Nach etwa 3h Inkubation bei 25°C werden chemokompetente Zellen von Escherichia coli K12 Stamm MC 1061 (Wertmann et al. Gene 49, 253-262, 1986) transformiert. Hierzu werden entsprechend vorbehandelte und bei -80°C gelagerte Zellen aufgetaut und zu beispielsweise 100µl Zellsuspension ein Aliquot des Ligationsansatzes zugegeben und durchmischt. Nach einer Inkubation für 15 min auf Eis, werden die Proben für 5 min bei 37°C in einem Wasserbad inkubiert und wieder auf Eis gestellt. Nun wird auf Agarplatten aus LB-Medium (0.5% w:v Bacto-Trypton, 1 % w:v Yeast-Extrakt, 0.1M NaCl) mit einem entsprechenden Antibiotikum (zumeist 200µg/ml Ampicillin) ausgespatelt. Die Platten werden über Nacht im Brutschrank bebrütet und nachfolgend einzelne Klone analysiert.

Für die analytische Isolierung von Plasmid-DNA durch alkalische Lyse (Birnboim & Doly, Nucl. Acids Res. 7, 1513-1523, 1979) nimmt man 1.5ml einer Übernachtkultur bei 37°C des zu charakterisierenden Klones in LB-Medium (mit Antibiotikum). Nach Pelletierung der E. coli Zellen wird der Überstand abgenommen, das Zellpellet in 200µl 10mM EDTA-Lsg. pH8 resuspendiert und nacheinander 400µl Denaturierungspuffer (0.2N NaOH, 1 % w:v SDS) und 300µl Kaliumacetat-Puffer (5M KAcO pH 4.7) zugegeben. Die Reaktionsgefäße werden geschüttelt und zur Pelletierung des Präzipitates zentrifugiert. Der Überstand wird in ein neues Reaktionsgefäß überführt und mit 500µl neutralem Phenol (Phenol äquilibiert in 0.1M Tris pH 7.5) extrahiert. Der Überstand nach Extraktion wird abgenommen und die enthaltene Plasmid-DNA mit Isopropanol präzipitiert. Die getrocknete DNA wird in 50µl TE Puffer mit RNAse (5mM Tris pH 8 0.1mM EDTA 20µg/ml RNAse) gelöst. Für die präparative Isolierung von DNA wird im Prinzip in ähnlicher Weise vorgegangen, allerdings mit entsprechend größeren Mengen. Zudem erfolgt die Aufreinigung der Plasmid-DNA durch Caesiumchlorid-Dichtegradienten-Zentrifugation. Die Quantifizierung isolierter Plasmid-DNA wird durch Aufnahme eines UV-Spektrums (Wellenlänge 220-300nm) vorgenommen. Dabei entspricht eine Abs₂₆₀ₙₘ = 1 einer Menge von 50µg doppelsträngiger DNA.

### Ergebnis:

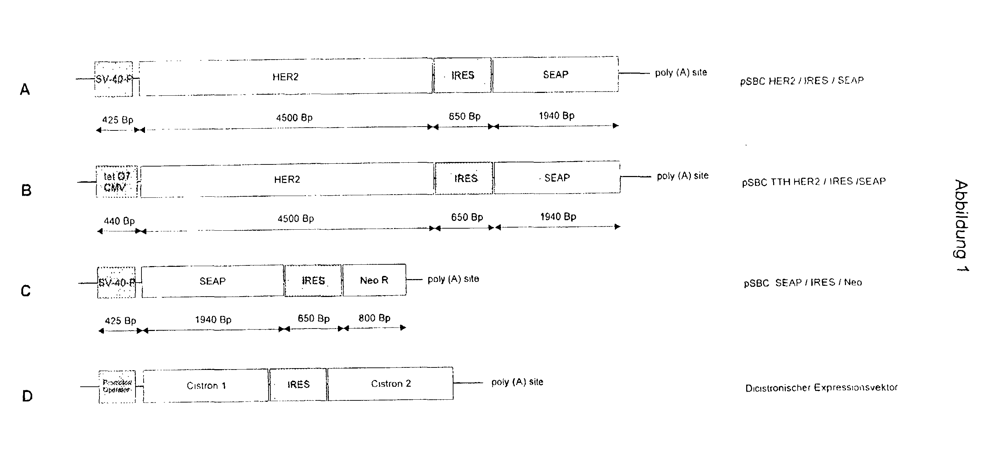

Die für die Klonierungsexperimente eingesetzten Vektoren pSBC1 und pSBC2 sowie entsprechende Derivate pSBC1 SEAP und pSBC2 SEAP wurden erstmals von Dirks et al. beschrieben (Gene 128, 247-249, 1993). Für die dicistronische Expression des HER2 Rezeptorproteins, wurde aus dem Vektor pCOB213 (Yamamoto et al., Nature 319, 230-234, 1986) über Verdau mit Mlul die HER2 cDNA isoliert und nach auffüllen mit Klenov-Polymerase in den mit Smal geöffneten Vektor pSBC1 ligiert. Das resultierende Konstrukt pSBC1 HER2 wurde über Verdau mit AseI / NotI mit pSBC2 SEAP kombiniert, wodurch das dicistronische Konstrukt pSBC HER2 / IRES / SEAP entsteht (Abb. 1A). In diesem Vektor ist die Expression des HER2 Proteins, welches nach Überexpression Fibroblasten transformiert, über die IRES Sequenz mit dem Reporter SEAP gekoppelt. Die Expression erfolgt konstitutiv unter Kontrolle des "immediate-early promotor" von SV-40.

Für das entsprechende Konstrukt mit einem durch Tetracyclin (oder Derivat) regulierbaren Promotor, wurde ein modifizierter Vektor pSBCTTH1 verwendet. Dieser Vektor enthält anstelle der SV-40 Promotors/Enhancer Sequenzen von pSBC1 die tetO₇ /CMV-Minimalpromotor Sequenz aus pUHC13-3 (Gossen & Bujard, Proc. Natl. Acad. Sci. USA 89, 5547-5551, 1992). Die Umklonierung der HER2 cDNA aus pSBC1 HER2 erfolgte über EcoRI / EcoRV / NotI in den EcoRI / NotI geöffneten Vektor pSBCTTH1 und resultiert im Konstrukt pSBCTTH1 HER2. Dieses wurde wie oben beschrieben über AseI/NotI mit pSBC2 SEAP kombiniert, woraus das induzierbare dicistronische Konstrukt pSBCTTH HER2 / IRES / SEAP resultiert (Abb. 1B).

Aus dem Vektor pAG60 (Colbère-Garapin et al. J. Mol. Biol. 150, 1-14, 1981), wurde mittels PCR aus der cDNA kodierend für das Enzym Aminoglykosid-3'phosphotransferase Typ2 die für das Protein kodierende Region amplifiziert. Das Enzym Aminoglykosid-3'-phosphotransferase vermittelt eine Resistenz gegen die Antibiotika Kanamycin oder Neomycin, aber auch gegen das u.a. für Säugerzellen toxische Gentamycin Derivat Geneticin (G418®). Durch die in den zur PCR verwendeten Oligonukleotid-Primern enthaltenen Schnittstellen EcoRI / Hindlll, wurde das Gen entsprechend in pSBC2 kloniert. Durch eine wie bereits oben beschriebene Umklonierung über AseI/NotI in den Vektor pSBC1 SEAP, entsteht das Konstrukt pSBC SEAP / IRES / Neo^{R} (Abb.1C).

Der Aufbau der für die im vorliegenden Patent verwendeten Vektoren ist in Abb. 1 zusammengefaßt dargestellt und beispielhaft zu verstehen. Es kann ein beliebiges transformierendes Gen in Cistron 1 - beispielsweise pSBC Onkogen / IRES / SEAP - oder ein beliebiges Resistenzgen in Cistron 2 - beispielsweise pSBC SEAP / IRES / Resistenzgen - plaziert werden (Abb. 1D). Entsprechend variabel ist das zur Transkription benötigte Promotor-/Operatorelement - konstitutiv oder induzierbar - zu verstehen.

### Beispiel 2: Hochsensitive Bestimmung der SEAP Aktivität

Nachfolgend sind die Methoden des Nachweises von SEAP-Aktivität aus Zellkulturüberständen in-vitro sowie aus Serum in-vivo beschrieben. Diese Methoden werden für die sensitive Detektion von SEAP verwandt. Zur Detektion wird aus humaner Placenta aufgereinigte Phosphatase (PLAP) definierter Aktivität verwendet.

### Methoden:

Entnahme von Serum aus Versuchstieren. Für die Bestimmung von SEAP-Aktivität im Serum von Nacktmäusen entnimmt man etwa 300µl Vollblut aus der Vena sublingualis. Das Vollblut läßt man 20 min bei 4°C gerinnen und zentrifugiert nachfolgend für 10 min bei 4°C in einer Heraeus Biofuge 15R bei 4500upm. Man nimmt den Überstand ab und zentrifugiert erneut für 3 min unter gleichen Bedingungen. Nach der zweiten Zentrifugation kann das Serum direkt zur Aktivitätsbestimmung verwendet oder bei -20°C gelagert werden. Entsprechend wird von in-vitro Zellkulturen zu definierten Zeitpunkten Zellkulturüberstand zwecks SEAP-Aktivitätsbestimmung entnommen. Eventuell enthaltene Zelltrümmer werden durch Zentrifugation für 5 min bei 4500upm in einer Heraeus Biofuge 15R pelletiert.

Phospha-Light® Chemilumineszenz-Reportergen-Assay (Bronstein et al. Biotechniques 17, 172-177, 1994). Zur chemoluminometrischen Bestimmung von SEAP Aktivität mit CSPD® als Substrat wird das Phospha-Light® Assay (Tropix Inc.) verwendet. Diese Methode ist hochsensitiv und zum Nachweis geringster Enzymmengen geeignet. Je nach Test werden 15µl Serum oder Zellkulturüberstand direkt zu 50µl Verdünnungs-Puffer in einer 96well Mikrotiterplatte für Lumineszens-Messungen (Dynatech) pipettiert, für 30min bei 65°C inkubiert und nachfolgend zum Abkühlen auf Eis gestellt. Nach Zugabe von 50µl Testpuffer wird für 5 min bei 30°C inkubiert. Durch Zugabe von 50µl Reaktions-Puffer wird die Messung gestartet (Zusammensetzung der Reaktionsmischung: 50mM NaHCO₃ pH 9.5, 10mM Homoarginin, 1 mM MgCl₂, EMERALD Amplifyer, CSPD-Substrat). Das verwendete Meßgerät ist ein Mikrotiterplatten-Luminometer Typ MicroLumat LB 96 P (EG & G Berthold). Das Meßintervall beträgt 3 min, es erfolgen 3 (6) Messungen. Das Maximum der Lichtemission ist etwa 6min (bei Messung von SEAP-Aktivität aus Serum nach etwa 15 min) nach Zugabe des Reaktions-Puffers erreicht.

SEAP-Aktivitätsmessung mittels p-Nitrophenylphosphat als Substrat. Das Assay mißt die Hydrolyse von p-Nitrophenylphosphat zu p-Nitrophenol (Absorptionsmaximum bei 405nm, pH 9.8). Zur Messung wird Zellkulturmedium oder Serum aus Nacktmäusen, wie oben beschrieben vorbehandelt, eingesetzt. 100µl Enzymlösung werden für 5 min bei 65°C im Wasserbad inkubiert und nachfolgend für 2min bei 15000upm in einer Heraeus Biofuge 15R zentrifugiert. Der Überstand wird zum Enzymtest eingesetzt, wobei ein typischer Reaktionsansatz für Mikrotiterplatten sich aus 150µl 1x SEAP- Puffer (1 M Diethanolamin pH 9.8, 0.5mM MgCl₂, 10mM Homoarginin), 50µl 2x SEAP-Puffer Puffer (2M Diethanolamin pH 9.8, 1.0 mM MgCl₂, 20mM Homoarginin) und 50µl Zellkulturmedium oder Serum zusammensetzt. Die Reaktion wird durch Zugabe von 25µl Substratlösung (120mM p-Nitrophenylphosphat in 1x SEAP-Puffer) gestartet. Die Messung der Absorption bei 405nm (25°C Raumtemperatur) erfolgt 5 min nach Zugabe des Substrates in einem Beckman Mikrotiterplatten-Fotometer, Typ Biomek Plate Reader.

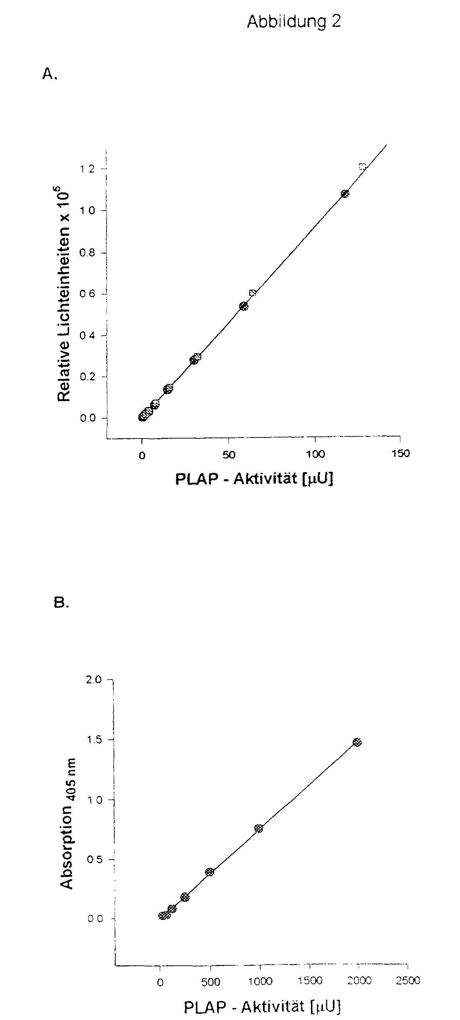

Zur Aufnahme entsprechender Standardkurven, wird aus humaner Placenta aufgereinigte alkalische Phosphatase definierter Aktivität (Calbiochem) unter den im Enzymtest verwendeten Versuchsbedingungen eingesetzt. Die Berechnung der Enzymaktivität in mUnits/ml erfolgt aus den aufgenommenen Standardkurven (siehe Abb.2), wobei eine "Unit" (abgekürzt U) definiert ist als die Menge von Enzym, die 1,0 µmol Paranitrophenylphosphat (PNPP) bei 30°C und pH 10,15 zu Paranitrophenol hydrolysiert.

### Ergebnis:

In Abb. 2 sind Ausschnitte der entsprechenden Eichkurven zur Bestimmung der Aktivität von SEAP aus Serum oder Zellkulturmedium mittels chemoluminometrischen (Abb. 2A) oder fotometrischen Assaysystemen (Abb. 2B) gezeigt. Der lineare Meßbereich des fotometrischen Enzymtestes liegt bei ≈100µUnits bis ≈2000µUnits PLAP. Die maximale Empfindlichkeit des Testes liegt unter Standardbedingungen bei etwa 100µUnits PLAP / ml, ist aber durch Erhöhung der Inkubationstemperatur und/oder Verlängerung der Meßzeiten noch zu verbessern. Der chemoluminometrische Enzymtest hat einen linearen Meßbereich von ≈ 0.06µUnits bis ≈ 500µUnits PLAP. Die maximale Empfindlichkeit unter Standardbedingungen liegt bei ≈ 0.4µUnits PLAP / ml. Zur Bestimmung der Enzymaktivität in Proben mit sehr hohen SEAP-Aktivitäten wird entsprechend im Inkubationsmedium verdünnt.

### Beispiel 3: Stabile Transfektion von NIH3T3 Fibroblasten

Nachfolgend sind die für die stabile Transfektion der NIH3T3 Zellinie (ATCC CRL 1658) verwendeten Methoden beschrieben, wobei ein von der genannten Zellinie ausgehend modifizierter Klon mit der Bezeichnung "NIH 3T3 Clone H.3" bei der Deutschen Sammlung von Mikroorganismen und Zellkulturen (DSM), D-38124 Braunschweig, unter der Nummer DSM ACC2242 hinterlegt wurde. Des weiteren ist die Analytik der hieraus resultierenden Zellinien auf die Transformations-spezifischen Parameter Focus- und Koloniebildung gezeigt.

### Methoden:

Stabile Transfektion mittels Lipofektion. Zur Transfektion von NIH3T3 Zellen wird das Lipofectamin®-Reagenz (Gibco BRL) verwendet. Die nachfolgend angegebenen Mengen beziehen sich auf eine 25 cm² Zellkulturflasche. Einen Tag vor der Transfektion werden die Zellen in einer Zelldichte von 1-3x10⁵ in normalem Kulturmedium (DMEM 4.5g/l Glucose, 10% v:v Kälberserum) gleichmäßig ausgesetzt (Dichte zum Zeitpunkt der Transfektion 50% - 80% Konfluenz). Für die Transfektion werden folgende Lösungen angesetzt: Lösung A: 6µg Kotransfer-DNA und 0.6µg Selektions-DNA in 300µl serumfreien Medium; Lösung B: 12-36µl Lipofectamin® in 300µl serumfreien Medium. Beide Lösungen werden gut vermischt und für 15min - 45min bei Raumtemperatur inkubiert, wobei sich der DNA-Liposomen-Komplex ausbildet. Je Ansatz werden nun 2.4ml serumfreies Medium zugegeben, während die Zellen mit 6ml serumfreien Mediums gewaschen werden. Nach Absaugen des gesamten Zellkulturmediums werden die Zellen mit dem DNA-Liposomen-Komplex überschichtet. Nach 5h Inkubation im Brutschrank bei 37°C und 5% CO₂ werden 3ml serumhaltiges Kulturmedium zugegeben. Am nächsten Tag werden die Zellen 1:20 bis 1:40 in frischem Kulturmedium passagiert. Am 3. Tage nach der Transfektion beginnt man mit der Selektion in Medium mit 125µg/ml Hygromycin B. Die Anzahl von Einzelzellklonen wird, sobald diese zu erkennen sind, im Phasenkontrast-Mikroskop bestimmt.

"Focus-Formation-Assay" für transfektierte NIH3T3 Fibroblasten (Riedel et al., Proc. Natl. Acad. Sci. USA 85, 1477-1481, 1988). Auf einer 8.5cm² Zellkulturplatte werden die zu testenden Zellen bis auf eine maximale Zelldichte kultiviert. Nach Erreichen einer konfluenten Zellschicht wird alle 3-4 Tage das Medium gewechselt, ohne dabei die Zellschicht zu zerstören. Zeigen sich Zellhaufen mit nicht mehr Kontaktinhibiert wachsenden Zellen ("Foci"), werden diese fixiert und durch Färbung mit Methylviolett angefärbt und gezählt. Alternativ können die Zellen nach Fixierung auch mit einem spezifischen monoklonalen Antikörper angefärbt werden. Für die Isolation der Zellen aus einem Focus verwendet man entsprechende Klonierungszylinder.

Zellwachstum in semisolidem Medium ("colony-assay"; Hamburger & Salmon, Science 197, 461-463, 1977). Der Test zur Bestimmung des Wachstums in semisolidem Medium besteht aus verschiedenen Agar-Schichten in 8.5cm² Kulturschalen: Schicht 1 besteht aus 1ml Zellkulturmedium mit 0.5% (w:v) Agar, Schicht 2 besteht aus 1 ml einer Monozellsuspension (2x10⁴ Zellen / ml) in Zellkulturmedium mit 0.25% (w:v) Agar; Schicht 3 besteht aus 1ml PBS und verhindert das Austrocknen des Agars. Die Inkubationsdauer im Brutschrank bei 37°C, 5% CO₂ beträgt etwa 21 Tage. Die Bestimmung der Koloniezahl erfolgt unter dem Binokular.

### Ergebnis:

Die Analyse verschiedener stabil transfizierter NIH3T3 Zellen mit u.a. den in Abb. 1 gezeigten Vektoren ist in Tabelle 1 zusammengefaßt. Die als Kontrolle ohne dicistronische Vektoren stabil transfizierten NIH3T3 Fibroblasten enthalten im Kulturmedium eine mit 0.01mU/ml geringe SEAP-Aktivität, die an der Nachweisgrenze des chemoluminometrischen Enzymtestes liegt. Die zur Kontrolle mit dem Konstrukt pSBCTTH SEAP transfizierten NIH3T3 Zellen sezernieren 3.18mU/ml SEAP in das Kulturmedium, bilden aber weder Foci noch Kolonien, d.h. sind nicht transformiert. Ein vergleichbares Ergebnis bezüglich der im Medium enthaltenen SEAP-Aktivität erhält man bei der Analyse von NIH3T3 Zellen, transfiziert mit dem Konstrukt pSBC HER2 / IRES /SEAP (9.30mU/ml). Allerdings sind die Zellen transformiert, wie sich im Focus-Bildungstest eindeutig zeigt.

Die Klonmischung von NIH3T3 Zellen, die mit dem induzierbaren dicistronischen Konstrukt pSBCTTH HER2 / IRES / SEAP transfektiert wurden, sezernieren 1.08mU/ml SEAP und bilden Foci, aber keine Kolonien aus. Ein aus einem Focus isolierter Einzelzellklon 12.3/G8 sezerniert 10.16mU/ml SEAP und zeigt sowohl Focusbildung als auch Kolonien in Weichagar, vergleichbar mit einem weiteren Einzelzellklon 12.3/H3, der 26.16mU/ml SEAP sezerniert. Die Regulation der Expression durch Tetracyclin (Konz. 1µg/ml) oder dessen Derivat Anhydrotetracyclin (Konz. 10ng/ml) in-vitro ist ebenfalls dargestellt. Die Expression des Reporters SEAP wird durch Zugabe der Antibiotika praktisch vollständig abgeschaltet, ebenso wird die Transformation revertiert (keine Kolonie- oder Focibildung). In Kontrollexperimenten wurde gezeigt, daß das Antibiotikum in den verwendeten Konzentrationen keinen wesentlichen antiproliferativen Effekt in-vitro besitzt.

In Abb.3 A/B ist die Expression der Rezeptortyrosinkinase HER2 mittels Nachweis durch den HER2-spezifischen monoklonalen Antikörpers 9G6 gezeigt (Phasenkontrast in Abb.3B, Fluoreszens in Abb.3B; Vergrößerung 100fach). Die HER2 Expression kann durch Anhydrotetracyclin abgeschaltet werden (Phasenkontrast in Abb.3C, Fluoreszens in Abb.3D; Vergrößerung 100fach). HER2 überexprimierende Zellklone im Foci-Assay (Abb.3 E/F; Vergrößerung 32/100fach) zeigen einen transformierten Phänotypus. Bei entsprechend durch Anhydrotetracyclin abgeschalteter HER2 Expression ist der transformierte Phänotypus revertiert (Abb.3 G/H; Vergrößerung 32/100fach).

Aus diesen Experimenten können folgende Schlußfolgerungen gezogen werden:
(i) HER-2 überexprimierende NIH3T3 Zellen sind transformiert
(ii) Die Expression des Reportergens SEAP oder die stabile Transfektion allein führen nicht zu einer Zelltransformation
(iii) HER-2 und SEAP werden gekoppelt in NIH3T3 Zellen exprimiert
(iv) Die Expression von HER2 und SEAP ist bei Verwendung des induzierbaren Promotors durch Kultur in Medium mit Tetracyclin oder einem Derivat hemmbar
(v) Die durch HER2 Expression induzierte Zelltransformation ist reversibel induzierbar

### Beispiel 4: Sensitiver Nachweis des "tumor-load" von HER 2 überexprimierenden NIH3T3 Fibroblasten mittels SEAP Aktivitätsbestimmung aus Serum

Für den Versuch werden Nacktmäuse verwendet, denen subkutan Zellen des NIH 3T3 Einzelzellklons 12.3/H3 implantiert wurden. Dieser Einzelzellklon wurde aus stabil mit dem Konstrukt PSBC TTH HER2/IRES/SEAP transfektierter NIH 3T3 Zellen isoliert. Gezeigt ist die Bildung des subkutanen Tumors als Funktion der Zeit mit den Meßparametern Tumormasse und SEAP-Aktivität im Serum der Versuchstiere.

### Methoden:

Tumorigenitätsprüfung an Nacktmäusen. Für den Tumorigenitätsnachweis verschiedener Zellinien anhand der Bildung subkutaner solider Tumore, werden weibliche Nacktmäuse des Stammes NMRI nu/nu verwendet. Nach einer 4 wöchigen Quarantänezeit werden die Tiere in Gruppen eingeteilt und durch Ohrlochung markiert. Zur Bestimmung der SEAP-Basalaktivität im Serum werden jedem Tier vor Versuchsbeginn ca. 300µl Vollblut aus der Vena sublingualis entnommen, hieraus das Serum gewonnen und die enthaltene SEAP-Aktivität bestimmt (siehe Beispiel 2). Die Zellimplantation erfolgt mittels subkutaner Injektion in die rechte Flanke des Versuchstieres. Appliziert werden beispielsweise 1 x 10⁶ Zellen (für Versuche mit NIH3T3 Zellen) in einem Volumen von 200µl PBS je Tier. Für die Applikation werden 1 ml Tuberkulinspritzen und 16er Kanülen verwendet. Während der Versuchsdauer von ca. 50 Tagen werden die Tiere zweimal wöchentlich untersucht. Hierbei wird das Körpergewicht bestimmt, eine Blutentnahme vorgenommen und palpatorisch das Tumorgewicht mittels Vergleich mit einem Standard-Plastilinmodell quantifiziert. Ist 50 Tage nach Zellimplantation noch kein Tumor tastbar, oder das Reportergen im Serum noch nicht nachweisbar, wird der Versuch beendet. Tiere mit exulcerierten Tumoren oder mit einem Tumorgewicht über 4g, sowie Tiere mit starken Körpergewichtsabnahmen oder schlechtem Allgemeinzustand, werden vorzeitig getötet. Getötete Tiere werden seziert und makroskopisch beurteilt. Organveränderungen, Tumorbeschaffenheit und eventuell gebildete Metastasen werden registriert. Das aus den Versuchstieren entnommene Tumorgewebe kann je nach Bedarf fixiert, in flüssigem Stickstoff schockgefroren oder für eine in-vitro Kultivierung aufgearbeitet werden.

### Ergebnis:

In Abb. 4A ist die im Serum nachweisbare SEAP Aktivität (in mU/ml) sowie die durch Tastung bestimmte Tumormasse (in g) gegen die Versuchszeit (VZ in Tagen) aufgetragen. In Kontrolltieren ist nur eine geringe hitzestabile Phosphatase Aktivität im Serum nachweisbar und wird von den Meßwerten subtrahiert. Aus Abb.4A wird deutlich, daß 3 Tage nach Injektion der Zellen, SEAP-Aktivität im Serum mit 4,66mU/ml (Mittelwert aus n=5) gut nachgewiesen werden kann. Ein tastbarer Tumor (≥ 0.1g) wird erst nach VZ 21 Tagen sichtbar (Abb.4B). Das Wachstum des Tumors wird bei allen Tieren aus der Zunahme der SEAP-Aktivität im Serum der Tiere deutlich. Die Korrelation des durch Tastung bestimmten Tumorgewichtes mit der im Serum gemessenen SEAP Aktivität ist in Abb.4C dargestellt und ist hoch signifikant. Aus diesem Versuch kann somit gefolgert werden, daß
(i) im Serum von Nacktmäusen eine hitzestabile Phosphatase-Aktivität nur in geringer Menge nachweisbar ist
(ii) von transfizierten, tumorigenen Zellen sezernierte SEAP in-vivo sensitiv aus entnommenem Serum meßbar ist
(iii) die aus dem Serum quantifizierte SEAP-Aktivität sehr gut mit dem Tumorgewicht korreliert und
(iv) eine Messung vitaler Tumorzellen möglich ist, selbst wenn noch kein Tumor tastbar ist

### Beispiel 5: Tetracyclin-regulierte Expression in gentechnisch veränderten Fibroblasten in-vivo

Für den Versuch werden Nacktmäuse verwendet, denen subkutan Zellen des NIH 3T3 Einzelzellklons 12.3/H3 implantiert wurden. Dieser Einzelzellklon wurde aus stabil mit dem Konstrukt PSBC TTH HER2/IRES/SEAP transfektierten NIH 3T3 Zellen isoliert. Gezeigt ist die Bildung des subkutanen Tumors als Funktion der Zeit mit den Meßparametern Tumormasse und SEAP-Aktivität im Serum der Versuchstiere. Zum VZ 28 bei Tastung von Tumoren ≥ 0.1g wurde den Tieren Anhydrotetracyclin injiziert, um die Expression von HER2 und SEAP transkriptionell abzuschalten.

### Methoden:

Behandlung der Tiere mit Anhydrotetracyclin. Für eine Behandlung im Rahmen eines Tumorigenitätstestes wird eine Dosis von 10 mg/kg dreimal pro Woche subkutan appliziert. Die LD₅₀ von Anhydrotetracyclin i.p. beträgt dabei 100mg/kg. Die Anhydrotetracyclin-Lösung wird in einer Konzentration von 1mg/ml in PBS angesetzt und aliquotiert bei -20°C gelagert. Je nach Fragestellung wird mit der Anhydrotetracyclin-Behandlung entweder direkt nach Zellimplantation oder erst nach Auftreten von Tumoren begonnen.

### Ergebnis:

Wie in Abb. 5 gezeigt ist, kann die Anzahl vitaler Tumorzellen über die SEAP-Aktivität im Serum sehr sensitiv quantifiziert werden und korreliert bis zum Zeitpunkt der Injektion von Anhydrotetracyclin (VZ 28) mit der durch Palpatation bestimmten Tumormasse. Dies entspricht den in Beispiel 4 gezeigten Ergebnissen. Durch die Injektion von Anhydrotetracyclin, wird die Transkription der dicistronischen mRNA abgeschaltet. Dies wird durch die lineare Abnahme der SEAP-Aktivität im Serum behandelter Tiere deutlich (Abb. 5A). Am VZ 38 ist mit 0.22mU/ml nur noch eine geringe, am VZ 45 ist bei allen Tieren die Aktivität hitzestabiler Phosphatase im Serum auf nicht mehr nachweisbare Mengen reduziert. Aus dem direkten Übergang und linearen Verlauf der beiden Kurven (SEAP-Aktivität vor / nach Behandlung) ist außerdem zu schließen, daß Anhydrotetracyclin sehr schnell und effektiv transkriptionell inhibiert. Aus dem linearen Verlauf der SEAP-Aktivität im Serum nach Injektion von Anhydrotetracyclin kann die Plasmahalbwertszeit von SEAP mit etwa 28h bestimmt werden. Dies ist für die Verwendung als Reporter für einen Therapieversuch von großer Bedeutung. In dem gezeigten Experiment wurde praktisch ein solches Therapieexperiment simuliert. Es kommt unter den gewählten Versuchsbedingungen und innerhalb der Versuchszeit von 45 Tagen zu einem Proliferationsstop. Das Tumorgewicht bleibt bei etwa 0.1g konstant. In Paralellexperimenten wurde mit der Injektion der gentechnisch veränderten Zellen auch gleichzeitig Anhydrotetracyclin injiziert. In diesen Versuchstieren sind nur marginale SEAP-Aktivitäten im Serum nachweisbar und in keinem Fall kommt es zur Ausbildung eines tastbaren Tumors.

### Beispiel 6: Stabile Transfektion verschiedener Tumorzellinien

Verschiedene Tumorzellinien, die für eine subkutane oder orthotope Transplantation auf der Nacktmaus geeignet sind, wurden auf hitzestabile Phosphatase Aktivität in Zellkulturüberständen getestet. Beispielhaft wurden die Tumorzellinien HT29 (ATCC HTB 38), KB (ATCC CCL17) und OVXF 899L stabil mit dem dicistronischen Konstrukt pSBC SEAP / IRES / Neo^{R} transfektiert, wobei ein von der Zellinie HT29 ausgehend modifizierter Klon mit der Bezeichnung "HT29 transfection H 20.2" bei der Deutschen Sammlung von Mikroorganismen und Zellkulturen, D-38124 Braunschweig, unter der Nr. DSM ACC2243 hinterlegt wurde, und die Aktivität an SEAP in Kulturüberständen bestimmt. Im einzelnen handelt es sich um die folgenden Tumorzellinien:
HT-29 (ATCC HTB 38; Fogh & Trempe, in: Human Tumor cells in-vitro, 115-159, Plenum Press, New York, 1975), eine Adenokarzinom-Zellinie des Colons
OVXF 899L (Licht et al. Proc. Amer. Assoc. Cancer Res. 31, A2210, 1990), eine Ovarialkarzinom Zellinie
SK-BR-3 (ATCC HTB 30), eine Zellinie aus einem Adenokarzinom der Brust
KB (ATCC CCL 17; Eagle, Proc. Soc. Exp. Biol. Med. 89, 362, 1955), eine Zellinie aus einem Karzinom der Mundschleimhaut
LXFA 629L, einem Adenokarzinom der Lunge
MEXF 514L (Licht et al., Journal of Cancer Research and Clinical Oncology, 118(2), 1992, 116-122), einer Zellinie aus einem malignen Melanom
AR42J (ATCC CRL1492), eine Zellinie aus einem Karzinom des exokrinen Pankreas der Ratte
GH3 (ATCC CCL 82.1; Endocrinology 82, 342-352, 1968), eine Zellinie aus einem Hypophysentumor der Ratte

Bis auf die beiden zuletzt genannten Zellinien AR42J und GH3 handelt es sich ausschließlich um Zellinien etabliert aus humanen Tumoren.

### Methoden:

Die Aktivitätsbestimmung von hitzestabiler Phosphatase erfolgte unter Verwendung der in Beispiel 2 genannten Enzymassays. Zur stabilen Transfektion wird beispielsweise die Calciumphosphat/DNA Kopräzipitation Methode (Wigler et al. Cell 1977, Vol.16, 777-785) oder die bereits in Beispiel 3 genannte Methode der Lipofektion verwendet. Nachfolgend ist die erstgenannte Methode beschrieben. Die angegebenen Mengen beziehen sich auf eine 25 cm² Zellkulturflasche.

Einen Tag vor der Transfektion werden die Zellen in einer Zelldichte von 1 - 3x10⁵/ 25cm² Kulturfläche gleichmäßig ausgesät. Am Tag der Transfektion wird 4h vor Zugabe des DNA-Präzipitates das Kulturmedium gewechselt. Das DNA-Präzipitat wird wie folgt hergestellt: In einem entsprechend dimensionierten Gefäß wird 250µl 2x HEBS-Puffer (280mM NaCl, 1.5mM Na₂HPO_{4,} 50mM Hepes pH 7.1) vorgelegt (0.5 vol 2xHEBS Puffer; Gesamtvolumen je Präzipitat sind 500µl). In ein weiteres Reaktionsgefäß pipettiert man 25µl 2.5M CaCl₂-Lösung (Endkonzentration 125mM) sowie die entsprechenden DNA, aufgeteilt nach Selektions-DNA (500ng), Cotransfer-DNA (5µg) und Carrier-DNA (5µg; gescheerte chromosomale DNA der zur Transfektion verwendeten Zellinie) und füllt auf ein Gesamtvolumen von 250µl mit Milli-Q Wasser auf. Nun gibt man unter Aufwirbeln des vorgelegten 2xHEBS Puffers das CaCl₂-DNA Gemisch zu diesem hinzu. Diese Mischung wird für 30 - 60 min bei Raumtemperatur belassen (Bildung des Präzipitates) und nachfolgend in das Kulturmedium zu den Zellen gegeben. Nach einer Inkubationszeit von 4-12h wird das Medium gewechselt. Mit der Selektion (Medium mit Selektionsmarker wie beispielsweise Geneticin/G418^{Ò}) beginnt man 2 Tage nach Beendigung der Transfektion.

### Ergebnis:

Die Bestimmungen der Phosphatase-Aktivität in den nicht-transfizierten Zellinien sind in Tab.2 zusammenfassend dargestellt. Danach wird von der KB und SK-BR-3 Tumorlinie eine signifikante Menge an hitzestabiler Phosphatase sezerniert, nämlich 4.1mU/ml bzw. 0.5mU/ml. Im Kulturmedium der Tumorlinie LXFA 629L ist mit 0.17mU/ml eine geringe Aktivität meßbar. Für die KB-Linie ist aus der Literatur bekannt, daß ein Dimer aus PLAP und IAP synthetisiert wird, der sich ähnlich wie PLAP verhält (Kodama et al., Biochem. Biophys. Acta 1218, 163-1172, 1994) und ganz offensichtlich auch sezerniert wird. Alle anderen Tumorzellinien sezernieren keine nachweisbaren Mengen an hitzestabiler Phosphatase (eine Aktivität von 0.01 mU/ml liegt an der Empfindlichkeitsgrenze des chemoluminometrischen Tests). Beispielhaft ist für drei Tumorzellinien gezeigt, daß nach Transfektion des entsprechenden Konstruktes pSBC SEAP / IRES / Neo^{R} im Kulturmedium hitzestabile AP-Aktivität nachweisbar ist. Diese ist in den Klonmischungen 4 bis 20fach höher als in den nicht-transfektierten Tumorzellen (siehe Tab. 2).

### Beispiel 7: Orthotope Transplantation der transfektierten Tumorzellinie HT29 in das Darmepithel von Nacktmäusen

Beispielhaft ist nachfolgend für die Tumorzellinie HT29 eine orthotope Transplantation in das Caecum von Nacktmäusen im Vergleich mit einer subkutanen Implantation gezeigt. Die für das Transplantationsexperiment verwendete HT29 Zellinie wurde hierzu stabil mit dem SEAP-cDNA Gen (Vektor pSBC SEAP / IRES / Neo^{R}) transfektiert.

### Methoden:

Orthotope Transplantation von HT29 Tumorzellen in das Caecum von Nacktmäusen. Zur Bestimmung der SEAP-Basalaktivität im Serum werden jedem Tier drei Tage vor Versuchsbeginn ca. 300µl Vollblut aus der Vena sublingualis entnommen, hieraus das Serum gewonnen und die enthaltene SEAP-Aktivität bestimmt (siehe Beispiel 2). Die Transplantation erfolgt unter einer Isofluran/Stickoxydul -Inhalationsnarkose. In Höhe des Kreuzbeinbereiches wird mit einer Präparierschere ein ca. 1 cm langer Hautschnitt durchgeführt und die Haut großzügig vom Unterhautgewebe gelöst. Entlang der Linea alba wird der Bauchraum eröffnet und mit einer Irispinzette das Caecum aus dem Bauchraum herausgezogen. Die zu transplantierenden Zellen werden resuspendiert und in einer 1 ml Tuberkulinspritze aufgezogen (1 x 10⁴Zellen / 50µl PBS / Tier). Die Zellinjektion in die Darmwand erfolgt zwischen Caecum und Ileumende. Anschließend wird der Darm vorsichtig reponiert, die Bauchdecke mit Catgut vernäht und der Hautschnitt mit Michelklammern verschlossen. Fünf Tage nach dem operativen Eingriff werden die Michelklammern entfernt. Die Blutentnahmen erfolgen wie bereits beschrieben zweimal pro Woche.

### Ergebnis:

In Abb. 6 sind die Ergebnisse für die stabil transfektierte HT29 Tumorzellinie dargestellt. In Abb. 6A/B ist die SEAP-Aktivität im Serum vergleichend mit der Tumormasse bei subkutaner Transplantation in Nacktmäusen dargestellt (n = 6). Wie schon bei den zuvor gezeigten Versuchsdaten wird deutlich, daß über die Messung der SEAP-Aktivität im Serum die vitalen Tumorzellen sehr sensitiv detektiert werden können. Die Zunahme der SEAP-Aktivität im Serum mit fortschreiten der Zeit korreliert mit der durch Tastung bestimmten Tumormasse. In Abb. 4C sind nun die SEAP-Aktivitäten im Serum von Tieren mit orthotop transplantierten Tumoren (n = 4) dargestellt. Auch wenn im Vergleich mit dem Versuch mit subkutaner Transplantation nur etwa 10fach niedrigere SEAP-Aktivitäten nachweisbar sind, ist ein kontinuierlicher Anstieg meßbar. Dieser reflektiert das Wachstum der Tumorzellen in der Darmwand, wie nach Biopsie der Tiere gezeigt werden konnte. Ein von außen tastbarer Tumor war während des Versuches nicht nachweisbar.

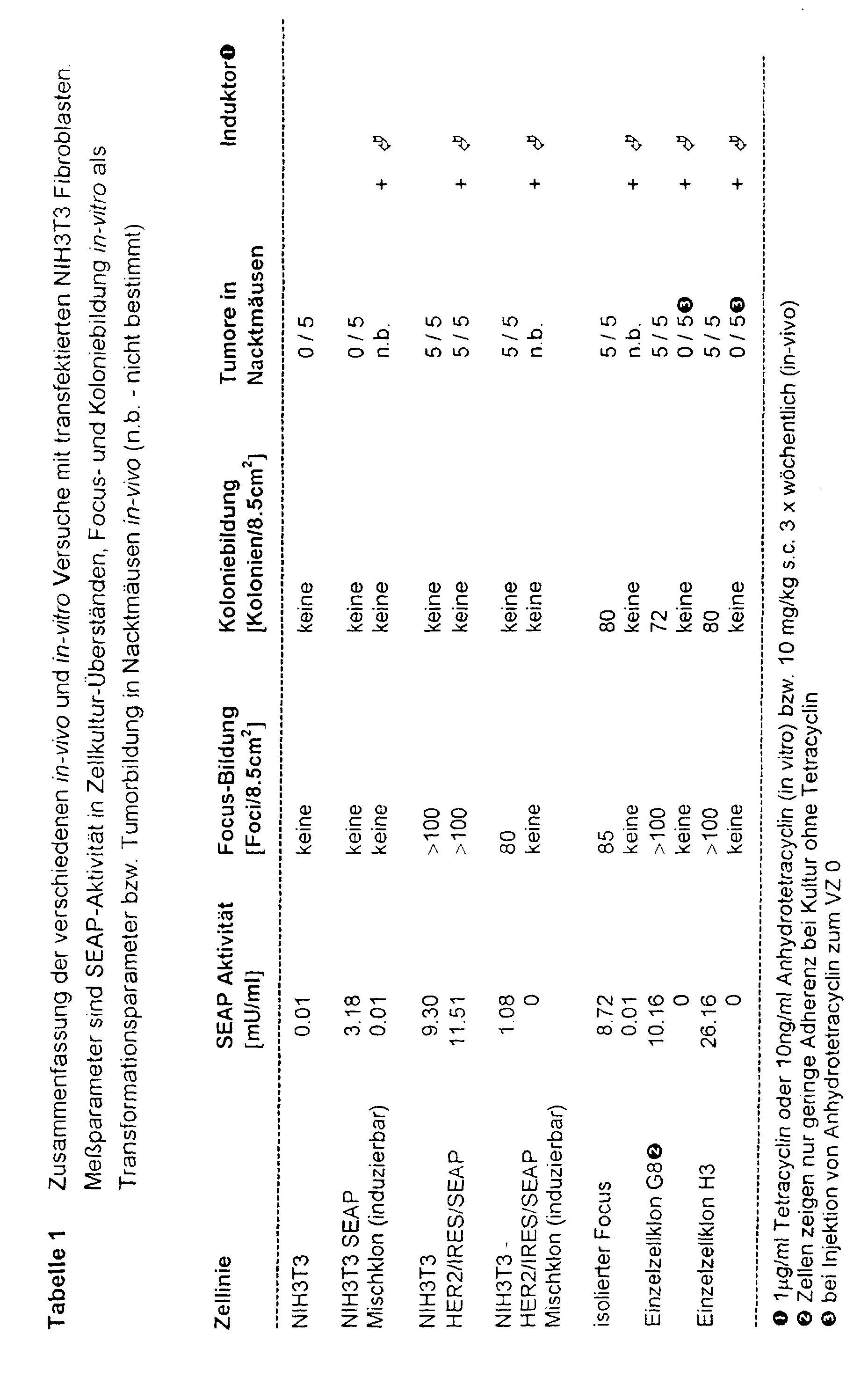

**Tabelle 2**

| Hitze-stabile alkalische Phosphatase Aktivität in Kulturüberständen von verschiedenen Tumorzellinien. Bis auf die Zellinien AR42J und GH3 sind diese ausschließlich humanen Ursprungs (n.b. - nicht bestimmt). | | |
|---|---|---|
| **Tumorzellinie** | **AP- Aktivität [mU/ml]** | **AP- Aktivität nach stabiler Transfektion [mU/ml]** |
| **HT29** (ATCC HTB38) Adenokarzinom des Kolons | ≈ 0.01 | 0.04 |
| **OVXF 899L** Ovarialkarzinom | ≈ 0.01 | 0.46 |
| **SK-BR-3** Adenokarzinom der Brust | 0.50 | n.b. |
| **KB** (ATCC CCL 17) Karzinom der Mundschleimhaut | 4.10 | 15.57 |
| **LXFA 629L** Adenokarzinom der Lunge | 0.17 | n.b. |
| **MEXF 514L** Melanom | ≈ 0.01 | n.b. |
| | | |
| **AR42J** (ATCC CRL 1492) Pankreaskarzinom der Ratte | 0.03 | n.b. |
| | | |
| **GH3** (ATCC CCL 82.1) Hypophysentumor der Ratte | ≈ 0.01 | n.b. |

### Abkürzungen:

- AP: alkalische Phosphatase
- ATP: Adenosin-5'-phosphat
- BSA: bovine serum albumin
- CMV: Cytomegalovirus
- CSPD: Disodium 3-(4-methoxyspiro{1,2-dioxetane-3,2'-(5'-chloro)tricyclo-[3.3.1.1³,⁷] decan}-4yl) phenylphosphate
- DNA: desoxyribonucleic acid
- DTT: Dithiothreitol
- EDTA: Ethylendiamintetraacetat
- ELISA: Enzyme linked immunosorbent assay
- HER: human EGF-receptor like
- IAP: Intestinal alkalische Phosphatase
- IRES: internal ribosomal entry site
- Neo^{R}: Gen kodierend für Aminoglykosid-3'-phosphotransferase
- PBS: phosphate buffered saline
- PLAP: humane Placenta-spezifische alkalische Phosphatase
- SDS: sodiumdodecylsulfate
- SEAP: sekretierte humane Placenta-spezifische alkalische Phosphatase
- SV-40: simian virus type 40
- tetO₇: Tetracyclin Operator
- tTA: Tetracyclin controlled transactivator
- U: Units
- upm: Umdrehungen pro Minute
- VZ: Versuchszeitpunkt in Tagen

## Patentansprüche

1. In-vivo-Verfahren zum Screening von Antitumor-Wirkstoffen in einem nichtmenschlichen Tiermodell, umfassend die Schritte:
a) Konstruktion eines Vektors, umfassend eine Nukleotidsequenz der allgemeinen Formel I
R - X - A - X - IRES - X - B - X - polyA (I)
in der
R eine regulatorische Nukleotidsequenz für eine konstitutive oder induzierbare Genexpression,
A ein Gen, kodierend für ein Protein, das ein tumorigenes Wachstum von Zellen induzieren kann,
IRES eine Nukleotidsequenz viralen, zellulären oder synthetischen Ursprungs, die in der Stufe der Translation für die interne Initiation verantwortlich ist,
B ein Gen, kodierend für ein sensitiv detektierbares sekretiertes Protein,
poly(A) eine Nukleotidsequenz für die Polyadenylierung des Transkriptes und
X optionale Linkersequenzen darstellen.
b) stabile Transfektion einer nicht tumorigenen Säugerzelllinie mit dem in Schritt a) erhaltenen Vektor,
c) Wachsen der in Schritt b) erhaltenen Säugerzelllinie in einem nicht-menschlichen Säugergewebe,
d) Entnahme eines Serums aus dem in Schritt c) erhaltenen nicht-menschlichen Säugetiergewebe und
e) Nachweis des Reporterproteins in dem Schritt d) erhaltenen Serum.

2. In-vivo-Verfahren zum Screening von Antitumor-Wirkstoffen in einem nicht-menschlichen Tiermodell, umfassend die Schritte:
a) Konstruktion eines Vektors, umfassend eine Nukleotidsequenz der allgemeinen Formel II
R - X - B -X - IRES - X - C - X - polyA (II)
in der
R eine regulatorische Nukleotidsequenz für eine konstitutive oder induzierbare Genexpression,
B ein Gen, kodierend für ein sensitiv detektierbares sekretiertes Protein,
IRES eine Nukleotidsequenz viralen, zellulären oder synthetischen Ursprungs, die in der Stufe der Translation für die interne Initiation verantwortlich ist,
C ein Gen, kodierend für ein Protein, das eine Selektion basierend auf einer Resistenz ermöglicht,
polyA eine Nukleotidsequenz für die Polyadenylierung des Transkriptes und
X optionale Linkersequenzen darstellen.
b) stabile Transfektion einer tumorigenen Säugerzelllinie mit dem in Schritt a) erhaltenen Vektor,
c) Wachsen der in Schritt b) erhaltenen Säugerzelllinie in einem nicht-menschlichen Säugergewebe,
d) Entnahme eines Serums aus dem in Schritt c) erhaltenen nicht-menschlichen Säugetiergewebe und
e) Nachweis des Reporterproteins in dem Schritt d) erhaltenen Serum.

3. Verfahren nach den Ansprüchen 1 und 2, worin R eine von SV-40 abgeleitete regulatorische Nukleotidsequenz ist.

4. Verfahren nach den Ansprüchen 1 und 2, worin R ein von SV-40 abgeleiteter Promotor ist.

5. Verfahren nach den Ansprüchen 1 und 2, worin R eine Tetracyclin-Operatorsequenz und einen Minimalpromotor umfaßt.

6. Verfahren nach den Ansprüchen 1 und 2, worin R die Nukleotidsequenz tetO₇CMV ist.

7. Verfahren nach den Ansprüchen 1 und 2, worin A ein für zelluläre Transformation kodierendes Gen ist.

8. Verfahren nach den Ansprüchen 1 und 2, worin A ein für eine Tyrosinkinase kodierendes Gen ist.

9. Verfahren nach den Ansprüchen 1 und 2, worin A ein für die Rezeptortyrosinkinase erbB2/HER2 kodierendes Gen ist.

10. Verfahren nach den Ansprüchen 1 und 2, worin B ein für eine sekretierte alkalische Phosphatase kodierendes Gen ist.

11. Verfahren nach den Ansprüchen 1 und 2, worin B ein für die sekretierbare humane Placenta-spezifische alkalische Phosphatase kodierendes Gen ist.

12. Verfahren nach den Ansprüchen 1 und 2, worin C ein für ein Enzym, das eine zelltoxische Verbindung inaktiviert, kodierendes Gen ist.

13. Verfahren nach Anspruch 12, worin C ein für Aminoglykosid-3'phosphotransferase kodierendes Gen ist.

## Claims

1. In vivo method for screening antitumour drugs in a non-human animal model, comprising the steps:
a) construction of a vector comprising a nucleotide sequence of the general formula I
R-X-A-X-IRES-X-B-X-polyA (I)
in which
R is a regulatory nucleotide sequence for a constitutive or inducible gene expression,
A is a gene coding for a protein which is able to induce tumorigenic growth of cells,
IRES is a nucleotide sequence of viral, cellular or synthetic origin which is responsible for internal initiation in the translation stage,
B is a gene coding for a sensitively detectable secreted protein,
poly(A) is a nucleotide sequence for polyadenylation of the transcript and
X are optional linker sequences.
b) stable transfection of a non-tumorigenic mammalian cell line with the vector obtained in step a),
c) growth of the mammalian cell line obtained in step b) in a non-human mammalian tissue,
d) removal of a serum from the non-human mammalian tissue obtained in step c) and
e) detection of the reporter protein in the serum obtained in step d).

2. In vivo method for screening antitumour drugs in a non-human animal model, comprising the steps:
a) construction of a vector comprising a nucleotide sequence of the general formula II
R-X-B-X-IRES-X-C-X-polyA (II)
in which
R is a regulatory nucleotide sequence for a constitutive or inducible gene expression,
B is a gene coding for a sensitively detectable secreted protein,
IRES is a nucleotide sequence of viral, cellular or synthetic origin which is responsible for internal initiation in the translation stage,
C is a gene coding for a protein which makes selection based on a resistance possible,
poly(A) is a nucleotide sequence for polyadenylation of the transcript and
X are optional linker sequences.
b) stable transfection of a tumorigenic mammalian cell line with the vector obtained in step a),
c) growth of the mammalian cell line obtained in step b) in a non-human mammalian tissue,
d) removal of a serum from the non-human mammalian tissue obtained in step c) and
e) detection of the reporter protein in the serum obtained in step d).

3. Method according to Claims 1 and 2, in which R is a regulatory nucleotide sequence derived from SV-40.

4. Method according to Claims 1 and 2, in which R is a promoter derived from SV-40.

5. Method according to Claims 1 and 2, in which R comprises a tetracycline operator sequence and a minimal promoter.

6. Method according to Claims 1 and 2, in which R is the nucleotide sequence tetO₇CMV.

7. Method according to Claims 1 and 2, in which A is a gene coding for cellular transformation.

8. Method according to Claims 1 and 2, in which A is a gene coding for a tyrosine kinase.

9. Method according to Claims 1 and 2, in which A is a gene coding for the receptor tyrosine kinase erbB2/HER2.

10. Method according to Claims 1 and 2, in which B is a gene coding for the secreted alkaline phosphatase.

11. Method according to Claims 1 and 2, in which B is a gene coding for the secretable human placenta-specific alkaline phosphatase.

12. Method according to Claims 1 and 2, in which C is a gene coding for an enzyme which inactivates a cytotoxic compound.

13. Method according to Claim 12, in which C is a gene coding for aminoglycoside 3'-phosphotransferase.

## Revendications

1. Procédé in vivo de criblage de substances actives anti-tumorales dans un modèle animal non humain, comprenant les étapes de :
a) construction d'un vecteur comprenant une séquence nucléotidique de formule générale (I)
R - X - A - X - IRES - X - B - X -polyA (I)
dans laquelle
R représente une séquence nucléotidique régulatrice pour une expression génétique constitutive ou inductible,
A représente un gène codant pour une protéine qui peut induire une croissance tumorigène de cellules,
IRES représente une séquence nucléotidique d'origine virale, cellulaire ou synthétique qui est à l'origine de l'initiation interne dans l'étape de traduction,
B est un gène codant pour une protéine sécrétée détectable de façon sensible,
Poly(A) représente une séquence nucléotidique pour la polyadénylation du produit de transcription et
X représente une séquence de lieur facultative,
b) transfection stable d'unee lignée cellulaire de mammifère non tumorigène avec le vecteur obtenu dans l'étape a),
c) croissance de la lignée cellulaire de mammifère obtenue dans l'étape b) dans un tissu de mammifère non humain,
d) prélèvement d'un sérum du tissu de mammifère non humain obtenu dans l'étape c) et
e) mise en évidence de la protéine rapporteuse dans le sérum obtenu dans l'étape d).

2. Procédé in vivo de criblage de substances actives antitumorales dans un modèle animal non humain, comprenant les étapes de
a) construction d'un vecteur qui comprend une séquence nucléotidique de formule générale (II)
R - X - B - X - IRES - X - C - X -polyA (II)
dans laquelle
R est une séquence nucléotidique régulatrice pour une expression génétique constitutive ou inductible,
B représente un gène codant pour une protéine sécrétrice détectable par voie sensible,
IRES représente une séquence nucléotidique d'origine virale, cellulaire ou synthétique, qui est à l'origine de l'initiation interne dans l'étape de traduction,
C est un gène codant pour une protéine qui permet une sélection sur la base d'une résistance,
PolyA est une séquence nucléotidique pour la polyadénylation du produit de transcription et
X représente des séquences de lieurs optionnels,
b) transfection stable d'une lignée cellulaires de mammifère tumorigène avec le vecteur obtenu dans l'étape a),
c) croissance de la lignée cellulaire de mammifère obtenue dans l'étape b) dans un tissu de mammifère,
d) prélèvement d'un sérum du tissu de mammifère obtenu dans l'étape c) et
e) mise en évidence de la protéine rapporteuse dans le sérum obtenu dans l'étape d).

3. Procédé selon les étapes 1 et 2, dans lesquelles R est une séquence nucléotidique régulatrice dérivant de SV-40.

4. Procédé selon les revendications 1 et 2, dans lequel R est un promoteur dérivé de SV-40.

5. Procédé selon les revendications 1 et 2, dans lequel R est une séquence opératrice de tétracycline et comprend un promoteur minimal.

6. Procédé selon les revendications 1 et 2, dans lequel R est la séquence nucléotidique tetO₇ cmV.

7. Procédé selon les revendications 1 et 2, dans lequel A est un gène codant pour une transformation cellulaire.

8. Procédé selon les revendications 1 et 2, dans lequel A représente un gène codant pour la tyrosine kinase.

9. Procédé selon les revendications 1 et 2, dans lesquelles A est un gène codant pour la tyrosine kinase réceptrice erbB2/HER2.

10. Procédé selon les revendications 1 et 2, dans lequel B représente un gène codant pour une phosphatase alcaline sécrétée.

11. Procédé selon les revendications 1 et 2, dans lequel B représente un gène codant pour la phosphatase alcaline spécifique de placenta humain sécrétable.

12. Procédé selon les revendications 1 et 2, dans lequel C représente une enzyme qui est un gène codant pour un composé toxique cellulaire inactivé.

13. Procédé selon la revendication 12 dans lequel C est un gène codant pour l'aminoglycoside-3'-phosphotransférase.
